# EUROPEAN PATENT SPECIFICATION

(11) **EP 2 097 527 B1**
(45) Date of publication and mention of the grant of the patent: **01.08.2018**
(21) Application number: 07817970.2
(22) Date of filing: 23.11.2007
(51) Int. Cl.: C12N 15/11

(54) **OLIGONUCLEOTIDES FOR MODULATING TARGET RNA ACTIVITY**
OLIGONUKLEOTIDE ZUR MODULATION VON ZIEL-RNA-AKTIVITÄT
OLIGONUCLÉOTIDES POUR MODULER L'ACTIVITÉ D'ARN CIBLE

(30) Priority: 23.11.2006 DK 200601543; 23.11.2006 DK 200601544; 04.02.2007 US 888094 P; 04.02.2007 US 888095 P; 24.07.2007 DK 200701081
(43) Date of publication of application: 09.09.2009
(73) Proprietor: Querdenker Aps, 2100 København (DK)
(72) Inventor: MØLLER, Thorleif, 216 14 Limhamn (SE)
(74) Representative: Orsnes, Henrik Egede
(86) International application number: PCT/DK2007/050174
(87) International publication number: WO 2008/061537

(56) References cited:
- WO-A-99/22772
- WO-A-2004/069992
- WO-A-2006/027776
- WO-A2-2007/112754
- US-A1- 2006 185 027
- SORRENTINO R. ET AL.: "Inhibition of MAPK activity, cell proliferation, and anchorage-independent growth by N-Ras antisense in an N-ras-transformed human cell line" ANTISENSE & NUCLEIC ACID DRUG DEVELOPMENT, vol. 11, 2001, pages 349-358, XP002496111
- JOHNSON S.M. ET AL.: "RAS is regulated by the let-7 microRNA family" CELL, vol. 120, 2005, pages 635-647, XP002496112
- KRÜTZFELDT J. ET AL.: "Strategies to determine the biological function of microRNAs" NATURE GENETICS, vol. 38, 2006, pages S14-S19, XP002496113
- Jiening Xiao ET AL: "Retracted: Novel approaches for gene-specific interference via manipulating actions of microRNAs: Examination on the pacemaker channel genes HCN2 and HCN4", Journal of Cellular Physiology, vol. 212, no. 2, 21 August 2007 (2007-08-21), pages 285-292, XP055199875, ISSN: 0021-9541, DOI: 10.1002/jcp.21062
- JIENING XIAO ET AL: "Retraction: Novel approaches for gene-specific interference via manipulating actions of microRNAs: Examination on the pacemaker channel genes HCN2 and HCN4", JOURNAL OF CELLULAR PHYSIOLOGY, vol. 227, no. 2, 25 February 2012 (2012-02-25), pages 877-877, XP055199876, ISSN: 0021-9541, DOI: 10.1002/jcp.23060
- W.-Y. Choi ET AL: "Target Protectors Reveal Dampening and Balancing of Nodal Agonist and Antagonist by miR-430", Science, vol. 318, no. 5848, 12 October 2007 (2007-10-12), pages 271-274, XP055199883, ISSN: 0036-8075, DOI: 10.1126/science.1147535
- CHOI WEN-YEE ET AL: "Target protectors reveal dampening and balancing of Nodal agonist and antagonist by miR-430.", SCIENCE (NEW YORK, N.Y.) 12 OCT 2007, vol. 318, no. 5848, 12 October 2007 (2007-10-12), pages 271-274, XP055199882, ISSN: 1095-9203
- GRUNWELLER A ET AL: "Locked nucleic acid oligonucleotides: The next generation of antisense agents?", BIODRUGS: CLINICAL IMMUNOTHERAPEUTICS, BIOPHARMACEUTICALS AND GENE THERAPY, ADIS INTERNATIONAL, FR, vol. 21, no. 4, 1 January 2007 (2007-01-01), pages 235-243, XP009125276, ISSN: 1173-8804
- M. Amarzguioui: "Tolerance for mutations and chemical modifications in a siRNA", Nucleic Acids Research, vol. 31, no. 2, 15 January 2003 (2003-01-15), pages 589-595, XP055408728, DOI: 10.1093/nar/gkg147
- SAYDA M ELBASHIR ET AL: "Functional anatomy of siRNAs for mediating efficient RNAi in Drosophila melanogaster embryo lysate", EMBO JOURNAL, OXFORD UNIVERSITY PRESS, SURREY, GB, vol. 20, no. 23, 3 December 2001 (2001-12-03), pages 6877-6888, XP002304126, ISSN: 0261-4189, DOI: 10.1093/EMBOJ/20.23.6877
- KURRECK J: "ANTISENSE TECHNOLOGIES IMPROVEMENT THROUGH NOVEL CHEMICAL MODIFICATIONS", EUROPEAN JOURNAL OF BIOCHEMI, WILEY-BLACKWELL PUBLISHING LTD, GB, vol. 270, no. 8, 1 April 2003 (2003-04-01) , pages 1628-1644, XP009045309, ISSN: 0014-2956, DOI: 10.1046/J.1432-1033.2003.03555.X
- BEREZIKOV EUGENE ET AL: "Approaches to microRNA discovery", NATURE GENETICS, NATURE PUBLISHING GROUP, NEW YORK, US, vol. 38 Suppl, 1 June 2006 (2006-06-01), pages S2-S7, XP002469102, ISSN: 1061-4036, DOI: 10.1038/NG1794

## Description

### Background of the invention

The present invention relates to oligonucleotides that can be used to affect the activity of target RNAs.

The first generation of such oligonucleotides were antisense oligonucleotides that were intended to affect the activity of target mRNAs. One reason for interest in such oligonucleotides is the potential for exquisite and predictable specificity that can be achieved because of specific base pairing. In other words, it is in theory very simple to design an oligonucleotide that is highly specific for a given nucleic acid, such as an mRNA.

However, it has turned out that not all sequences are available for antisense targeting and accessibility may vary e.g. because of secondary structure or protein binding.

Moreover, it has turned out simple base pairing is not enough to achieve regulation of a given target mRNA, i.e. an oligonucleotide complementary to a given target mRNA does not necessarily affect the activity of the target mRNA. If the oligonucleotide targets the open reading frame of an mRNA, it may e.g. be that the translational apparatus simply displaces the oligonucleotide during translation. Therefore, means where developed that would improve the regulatory activity of the oligonucleotide.

E.g. oligonucleotides that can activate RNase H cleavage of the target mRNA were developed. One potential disadvantage of such oligonucleotides is that they may mediate cleavage of other RNAs than the intended target mRNA, i.e. giving rise to off-target effects. Still, oligonucleotides acting through RNase H cleavage are in clinical trials for treatment of various diseases.

Recently, research has shown that eukaryotic cells, including mammalian cells, comprise a complex gene regulatory system (herein also termed RNAi machinery) that uses RNA as specificity determinants. This system can be triggered by so called siRNAs that may be introduced into a cell of interest to regulate the activity of a target mRNA. Currently, massive efforts go into triggering the RNAi machinery with siRNAs for specific regulation of target RNAs, in particular target mRNAs. This approach is widely regarded as having great promise for the development of new therapeutics. As will also be outlined below, a major advantage of this approach is that specificity of the siRNA lies in the degree of complementarity between the guide strand of the siRNA and the target RNA, i.e. target specificity can be controlled. However, it has turned out that siRNAs may be less specific than initially thought. Initially, it was believed that only target RNAs that harboured stretches of complete complementarity to the guide strand of the siRNA would be affected, i.e. targeted by the RNAi machinery. New research indicates that siRNAs indeed do result in significant off-target effects, i.e. regulation of non-intended targets. It is now believed that these off-targets stem from the siRNAs, or rather the guide strand of the siRNAs, acting as microRNAs.

MicroRNAs are a class of endogenous RNA molecules that has recently been discovered and that, as siRNA, function via the RNAi machinery. Currently, about 500 human microRNAs have been discovered and the number is rapidly increasing. It is now believed that more than one third of all human genes may be regulated by microRNAs. Therefore, microRNAs themselves may be used to regulate the activity of target RNAs, and consequently e.g. be used as therapeutics.

However, as also described below, microRNAs generally act at more than one target RNA, i.e. they are promiscuous. Thus, introduction of a microRNA into the cell or regulating the level of a microRNA will affect the activity of more than one target mRNA and consequently may give rise to undesired off-target effects.

A recent approach has been put forward, wherein the activity of a target RNA is regulated by inhibiting the activity of a microRNA. The microRNA can be inhibited using complementary oligonucleotides that have been termed antimirs and antagomirs. Since the microRNA is itself promiscuous, also an antimir or antagomir will be promiscuous and affect the activity of more than one target RNA.

US 2006/185027 relates generally to methods for identifying microRNA targets. US 2006/185027 also includes a general disclosure that one could use an oligonucleotide that can bind to the mRNA target sequence or the microRNA sequence in order to block the ability of the microRNA to bind to the mRNA in order to increase gene expression. US 2006/185027 made no distinction between directly blocking a microRNA by binding to the microRNA and indirectly blocking a microRNA by binding to a microRNA binding site in an mRNA and provides no information as to how to design an oligonucleotide for increasing gene expression.

### Detailed description

It is to be understood that features described in one aspect of the invention equally applies for the other aspects.

### Definitions

An "oligonucleotide capable of regulating the activity of a target RNA" refers to an oligonucleotide with a particular activity. Such oligonucleotides are also termed active oligonucleotides.

The terms "regulate" and "modulate" are used interchangeably herein.

An "oligonucleotide potentially capable of regulating the activity of a target mRNA" refers to an oligonucleotide which activity has not yet been experimentally confirmed. Such an oligonucleotide may also be termed a candidate regulator.

When reference is made to an oligonucleotide without further specification, the oligonucleotide may be an "oligonucleotide capable of regulating the activity of an mRNA" or an "oligonucleotide potentially capable of regulating the activity of a target mRNA" or both.

When referring to a "target RNA", what is meant is the target for an oligonucleotide of the invention. Typically, an oligonucleotide of the invention can interact with a target RNA by way of base pairing.

The target RNA is a mRNA .

When referring to the "activity of a target mRNA", what is typically meant is the expression of the target mRNA, i.e. translation into a protein or peptide. Thus, regulation of the activity of a target mRNA may include degradation of the mRNA and/or translational regulation. Regulation may also include affecting intracellular transport of the mRNA. In a preferred embodiment of the invention, the oligonucleotide is capable of regulating the expression of the target mRNA. In another preferred embodiment, the oligonucleotide may mediate degradation of the target mRNA (in turn also regulating expression of the target mRNA). The activity may also be replication.

As used herein, regulation may be either positive or negative. I.e. a regulator (e.g. oligonucleotide or microRNA) may increase the activity of the target (e.g. target mRNA) or it may decrease the activity of the target.

When referring to the "target sequence of an RNA", what is meant is the region of the RNA involved in or necessary for microRNA regulation. The terms "target region" and "target sequence" are used interchangeably herein.

Not intended to be bound by theory, it is believed that this region comprise bases that interact directly with the microRNA during microRNA regulation of the target RNA. In a preferred embodiment, the target sequence is the region of the target RNA necessary for microRNA regulation. Such region may be defined using a reporter system, wherein systematic deletions of the target RNA are tested for activity to define the target sequence. Assessing the effect of introducing point mutations in the target region is also valuable for defining the target region.

As will be clear from the specification, also oligonucleotides of the invention may be used to define the region of the target RNA necessary for microRNA regulation.

Preferably, the target sequence comprises an antiseed sequence, which is complementary to the seed sequence of a microRNA and also complementary to a guide sequence of an oligonucleotide of the invention. Introduction of mutations in the antiseed sequence will typically affect microRNA regulation and hence may be used to verify that given positions are involved in microRNA regulation.

The term microRNA as used herein has the same meaning as typically in the art. I.e. the term microRNA refers to a small non-translated RNA of typically 18-22 nucleotides that is capable of regulating the activity of a target mRNA. A microRNA is typically processed from pri-microRNA to short stem-loop structures called pre-microRNA and finally to mature miRNA. Both strands of the stem of the pre-microRNA may be processed to a mature microRNA.

The miRBase (http://microrna.sanger.ac.uk/sequences/) is a compilation of known microRNAs. Also predicted and known targets of the microRNAs can be found on this site.

The term siRNA (short interfering RNA) as used herein has the same meaning as typically in the art. I.e. the term siRNA refers to double stranded RNA complex wherein the strands are typically 18-22 nucleotides in length. Very often, the complex has 3'-overhangs.

When referring to the RNAi machinery herein, what is meant are the cellular components necessary for the activity of siRNAs and microRNAs or for the RNAi pathway. A major player of the RNAi machinery is the RNA induced silencing complex (the RISC complex).

As referred to herein, a RNA unit is one of the monomers that make up an RNA polymer. Thus, an RNA unit is also referred to as an RNA monomer or a RNA nucleotide. Likewise, a DNA unit is one of the monomers that make up a DNA polymer and a DNA unit may also be referred to as a DNA monomer or a DNA nucleotide.

When referring to a base, what is meant is the base of a nucleotide. The base may be part of DNA, RNA, INA, LNA or any other nucleic acid or nucleic acid capable of specific base pairing. The base may also be part of PNA (peptide nucleic acid). In some embodiments, the base may be a universal base.

When referring the length of a sequence, reference may be made to the number of units or to the number of bases.

When referring to a complementary sequence, G pairs to C, A pairs to T and U and vice versa. In a preferred embodiment, G also pairs to U and vice versa to form a so-called wobble base pair. In another preferred embodiment, the base inosine (I) may be comprised within either in a microRNA or oligonucleotide of the invention. I basepairs to A, C and U. In still another preferred embodiment, universal bases may be used. Universal bases can typically basepair to G, C, A, U and T. Often universal bases do not form hydrogen bonds with the opposing base on the other strand. In still another preferred embodiment, a complementary sequence refers to a contiguous sequence exclusively of Watson-Crick base pairs.

### Summary of the invention

In a first aspect, the present invention provides a method of providing an oligonucleotide for blocking the regulatory activity of a microRNA at a target mRNA. In another aspect, the invention provides an oligonucleotide for blocking the regulatory activity of a microRNA at a target mRNA. In yet another aspect, the invention provides an ex vivo or in vitro method of modulating the activity of a target mRNA by preventing the activity of a microRNA at the target mRNA.

### Disclosure of the invention

### First aspect - bioactive oligonucleotides

In one aspect, the present invention provides an oligonucleotide comprising an antisense sequence that comprises a guide sequence corresponding to the seed sequence of a microRNA.

Such an oligonucleotide is of interest because it can be used to target the target region of a target RNA, said target region being involved in microRNA regulation of the target RNA. Not intended to be bound by theory, it is believed that said target region will be more accessible for interaction (with microRNAs, oligonucleotides or other nucleic acids) than will other regions of the target RNA, because the target region is evolved for interaction with a microRNA or because endogenous microRNAs chooses target regions that are more accessible.

Support for the above view comes from work published after the priority date of this patent application. One publication investigated the effect of target secondary structure on the efficacy of repression by microRNAs (Long D, 2007). The results indicate a potent effect of target structure on target recognition by microRNAs, at least for microRNA regulation in Caenorhabditis elegans and Drosophila melanogaster. The authors suggest that target secondary structure probably contributes to accessibility in most miRNA-target interactions.

Another study systematically investigated the role of target-site accessibility in microRNA target recognition (Kertesz M, 2007). The authors demonstrated that mutations diminishing target accessibility substantially reduce microRNA mediated translational repression. Moreover, the authors performed a genome-wide analysis of target accessibility to all 3'UTRs of fly, worm, mouse and human. They found that microRNA seed sequences in all four organisms showed a notable preference for highly accessible regions and the authors suggest that target accessibility is a critical factor in microRNA function.

We suggest that target accessibility will most likely be determined by a combination of target secondary structure and occlusion by other factors such as RNA binding proteins.

As used in the context of the guide sequence (of an oligonucleotide of the invention) and the seed sequence (of a microRNA), the word "corresponding" refers to the ability of the seed sequence and the guide sequence of being capable of base pairing with the same sequence. I.e. the guide sequence and the seed sequence may not necessarily be identical, but they are capable of base pairing to the same sequence, e.g. the anti-seed sequence of a target RNA.

The phrase "a sequence corresponding to the complete sequence of a microRNA sequence" is intended to cover e.g. a precursor of the microRNA or a DNA molecule that encode the microRNA. The DNA molecule may e.g. be a PCR product intended for T7 RNA polymerase transcription of the microRNA. Such molecules are not included in the scope of the invention, as neither are naturally occurring microRNAs.

### Origin

The target RNAs to be used in the methods of the present invention are preferably of human origin. The target RNAs may also be of viral origin, preferably from virus that infects humans. In a preferred embodiment, the term human target RNA also includes viral target RNAs of virus that infects humans.

The microRNAs to be used in the methods of the present invention are also preferably of animal or plant origin. More preferably, they are of mammalian origin. Most preferably, they are of human origin. The microRNAs to be used in the methods of the present invention may also be of viral origin. If they are of viral origin, they are preferably from virus that infects humans, e.g. mir-LAT of HSV-1. In a preferred embodiment, the term human microRNAs also include viral microRNAs of virus that infect humans.

It is preferred that the oligonucleotides of the invention comprise a guide sequence that corresponds to the seed sequence of a human microRNA or of a microRNA from a virus that infects humans.

In a preferred embodiment, the oligonucleotide of the invention comprise a sequence that is capable of base pairing to the complementary sequence of position 2-7of any SEQ ID NOs:1-313.

The term complementary sequence has been defined above. The phrase "are capable of base pairing to" is related to the term complementary sequence. I.e. a first sequence is capable of base pairing to a second sequence, which is complementary to the first sequence.

### Contiguous stretch of bases

Preferably, the oligonucleotide of the invention comprises a antisense sequence that comprises a contiguous stretch of bases, complementary to the micro RNA target sequence of a target RNA of less than 35 bases and more than 7 bases.

A contiguous stretch of bases is intended to mean a non-interrupted sequence of bases that all fit into a duplex formed between the oligonucleotide of the invention and the target RNA. I.e. there are preferably no bulges in the duplex and it is preferred that the sequences are complementary (see the definition of complementary sequences above). Most preferred is perfect Watson-Crick duplex between the oligonucleotide of the invention and target region of the target RNA.

The terms contiguous and continuous are used interchangeably herein.

Preferably, the oligonucleotide can interact with the same region of the target RNA as a microRNA. One advantage of such an oligonucleotide is that it targets an exposed region of the target RNA (see discussion above). Another advantage of such an oligonucleotide is that is can be used to mask the microRNA target such that the (endogenous) microRNA targeting the target RNA will be prevented from interacting with the target RNA, and thus exert its effects on the target RNA.

The oligonucleotide of the invention has a degree of identity to its corresponding microRNA selected from the group consisting of less than 90. When referring to the degree of identity, the degree is counted over the length of the shortest molecule of the micro RNA and the oligonucleotide of the invention. The guide sequence of the oligonucleotide of the invention and the seed sequence of the microRNA is used for alignment. Hence, if the microRNA is 20 bases and the oligonucleotide is 14 and the number of identical positions are 12, the degree of identity is 12/14 = 86%. If the microRNA is 20, the oligonucleotide 20 and the number of positions is 10, then the degree of identity is 10/20 = 50%.

Preferably, the position of the guide sequence within the oligonucleotide of the invention is selected from the group consisting of: position 1, position 2, position 3, position 4, position 5, position 6, position 7, position 8, position 9, position 10, position 11, position 12, position 13, position 14, position 15, position 16, position 17, position 18 and position 19, wherein the position is counted in the 5'-3' direction from the first base of the guide sequence and the first base of the oligonucleotide.

More preferably the position is selected from the group consisting of: position 1, position 2, position 3, position 4 and position 5.

As mentioned earlier, the guide sequence corresponds to the seed sequence of a microRNA, which is defined elsewhere in the specification.

The length of the oligonucleotide of the invention may be adjusted for various purposes. A stronger interaction with the target RNA may be achieved by increasing the length of the oligonucleotide, as well as the stretch of bases complementary to the micro RNA target sequence of a target RNA. On the other hand, the length may be decreased for better delivery and bioavailability. A reduced length will give a decreased tm value (melting temperature) of the oligonucleotide. However, increasing the concentration of the oligonucleotide may be used to counteract this. Also, affinity increasing nucleotides and affinity increasing modifications may be used.

In a preferred embodiment, the length of the oligonucleotide is selected from the group consisting of: less than 60 bases, less than 50 bases, less than 40 bases, less than 39 bases, less than 38 bases, less than 37 bases, less than 36 bases, less than 35, less than 34 bases, less than 33 bases, less than 32 bases, less than 31 bases, bases, less than 30 bases, less than 29 bases, less than 28 bases, less than 27 bases, less than 26 bases, less than 25 bases, less than 24 bases, less than 23 bases, less than 22 bases, less than 21 bases, less than 20 bases, less than 19 bases, less than 18 bases, less than 17 bases, less than 16 bases, less than 15 bases, less than 14 bases, less than 13 bases, less than 12 bases, less than 11 bases, less than 10 bases, less than 9 bases, less than 8 bases, less than 7 bases, more than 60 bases, more than 50 bases, more than 40 bases, more than 39 bases, more than 38 bases, more than 37 more, more than 36 bases, more than 35, more than 34 bases, more than 33 bases, more than 32 bases, more than 31, more than 30 bases, more than 29 bases, more than 28 bases, more than 27 bases, more than 26 bases, more than 25 bases, more than 24 bases, more than 23 bases, more than 22 bases, more than 21 bases, more than 20 bases, more than 19 bases, more than 18 bases, more than 17 bases, more than 16 bases, more than 15 bases, more than 14 bases, more than 13 bases, more than 12 bases, more than 11 bases, more than 10 bases, more than 9 bases, more than 8 bases, more than 7 bases, more than 6 bases and more than 5 bases.

In another preferred embodiment, the length of the oligonucleotide is selected from the group consisting of between 10 and 14 bases, between 12 and 16 bases, between 14 and 18 bases, between 16 and 20, between 10 and 25 bases, between 12 and 24 bases, between 14 and 22 bases, between 15 and and 22 bases and between 15 and 20 bases.

More preferred is a length between 8 and 25 bases.

Most preferred is a length between 10 and 20 bases.

In a preferred embodiment of the invention, the microRNA has a sequence selected from the group consisting of SEQ NO:1-313.

Preferred microRNAs are listed in table 2.

**Table 2. A list of human microRNAs. Sequences are shown from the 5' to 3' direction.**

| **Name** | **Sequence** | **SEQ ID NO** |
|---|---|---|
| hsa-let-7a | UGAGGUAGUAGGUUGUAUAGUU | 1 |
| hsa-let-7b | UGAGGUAGUAGGUUGUGUGGUU | 2 |
| hsa-let-7c | UGAGGUAGUAGGUUGUAUGGUU | 3 |
| hsa-let-7f | UGAGGUAGUAGAUUGUAUAGUU | 4 |
| hsa-miR-100 | AACCCGUAGAUCCGAACUUGUG | 5 |
| hsa-miR-103 | AGCAGCAUUGUACAGGGCUAUGA | 6 |
| hsa-miR-106b | UAAAGUGCUGACAGUGCAGAU | 7 |
| hsa-miR-107 | AGCAGCAUUGUACAGGGCUAUCA | 8 |
| hsa-miR-10a | UACCCUGUAGAUCCGAAUUUGUG | 9 |
| hsa-miR-125b | UCCCUGAGACCCUAACUUGUGA | 10 |
| hsa-miR-126* | CAUUAUUACUUUUGGUACGCG | 11 |
| hsa-miR-127 | UCGGAUCCGUCUGAGCUUGGCU | 12 |
| hsa-miR-129 | CUUUUUGCGGUCUGGGCUUGC | 13 |
| hsa-miR-130a | CAGUGCAAUGUUAAAAGGGCAU | 14 |
| hsa-miR-130b | CAGUGCAAUGAUGAAAGGGCAU | 15 |
| hsa-miR-132 | UAACAGUCUACAGCCAUGGUCG | 16 |
| hsa-miR-135a | UAUGGCUUUUUAUUCCUAUGUGA | 17 |
| hsa-miR-136 | ACUCCAUUUGUUUUGAUGAUGGA | 18 |
| hsa-miR-141 | UAACACUGUCUGGUAAAGAUGG | 19 |
| hsa-miR-142-3p | UGUAGUGUUUCCUACUUUAUGGA | 20 |
| hsa-miR-146a | UGAGAACUGAAUUCCAUGGGUU | 21 |
| hsa-miR-146b | UGAGAACUGAAUUCCAUAGGCU | 22 |
| hsa-miR-147 | GUGUGUGGAAAUGCUUCUGC | 23 |
| hsa-miR-148a | UCAGUGCACUACAGAACUUUGU | 24 |
| hsa-miR-148b | UCAGUGCAUCACAGAACUUUGU | 25 |
| hsa-miR-150 | UCUCCCAACCCUUGUACCAGUG | 26 |
| hsa-miR-154 | UAGGUUAUCCGUGUUGCCUUCG | 27 |
| hsa-miR-154* | AAUCAUACACGGUUGACCUAUU | 28 |
| hsa-miR-15a | UAGCAGCACAUAAUGGUUUGUG | 29 |
| hsa-miR-15b | UAGCAGCACAUCAUGGUUUACA | 30 |
| hsa-miR-16 | UAGCAGCACGUAAAUAUUGGCG | 31 |
| hsa-miR-181a | AACAUUCAACGCUGUCGGUGAGU | 32 |
| hsa-miR-181a* | ACCAUCGACCGUUGAUUGUACC | 33 |
| hsa-miR-181c | AACAUUCAACCUGUCGGUGAGU | 34 |
| hsa-miR-182* | UGGUUCUAGACUUGCCAACUA | 35 |
| hsa-miR-184 | UGGACGGAGAACUGAUAAGGGU | 36 |
| hsa-miR-190 | UGAUAUGUUUGAUAUAUUAGGU | 37 |
| hsa-miR-191* | GCUGCGCUUGGAUUUCGUCCCC | 38 |
| hsa-miR-192 | CUGACCUAUGAAUUGACAGCC | 39 |
| hsa-miR-194 | UGUAACAGCAACUCCAUGUGGA | 40 |
| hsa-miR-195 | UAGCAGCACAGAAAUAUUGGC | 41 |
| hsa-miR-197 | UUCACCACCUUCUCCACCCAGC | 42 |
| hsa-miR-199a | CCCAGUGUUCAGACUACCUGUUC | 43 |
| hsa-miR-199b | CCCAGUGUUUAGACUAUCUGUUC | 44 |
| hsa-miR-19a | UGUGCAAAUCUAUGCAAAACUGA | 45 |
| hsa-miR-19b | UGUGCAAAUCCAUGCAAAACUGA | 46 |
| hsa-miR-200a | UAACACUGUCUGGUAACGAUGU | 47 |
| hsa-miR-200a* | CAUCUUACCGGACAGUGCUGGA | 48 |
| hsa-miR-203 | GUGAAAUGUUUAGGACCACUAG | 49 |
| hsa-miR-204 | UUCCCUUUGUCAUCCUAUGCCU | 50 |
| hsa-miR-205 | UCCUUCAUUCCACCGGAGUCUG | 51 |
| hsa-miR-206 | UGGAAUGUAAGGAAGUGUGUGG | 52 |
| hsa-miR-208 | AUAAGACGAGCAAAAAGCUUGU | 53 |
| hsa-miR-20a | UAAAGUGCUUAUAGUGCAGGUAG | 54 |
| hsa-miR-20b | CAAAGUGCUCAUAGUGCAGGUAG | 55 |
| hsa-miR-21 | UAGCUUAUCAGACUGAUGUUGA | 56 |
| hsa-miR-210 | CUGUGCGUGUGACAGCGGCUGA | 57 |
| hsa-miR-211 | UUCCCUUUGUCAUCCUUCGCCU | 58 |
| hsa-miR-212 | UAACAGUCUCCAGUCACGGCC | 59 |
| hsa-miR-215 | AUGACCUAUGAAUUGACAGAC | 60 |
| hsa-miR-218 | UUGUGCUUGAUCUAACCAUGU | 61 |
| hsa-miR-219 | UGAUUGUCCAAACGCAAUUCU | 62 |
| hsa-miR-22 | AAGCUGCCAGUUGAAGAACUGU | 63 |
| hsa-miR-220 | CCACACCGUAUCUGACACUUU | 64 |
| hsa-miR-221 | AGCUACAUUGUCUGCUGGGUUUC | 65 |
| hsa-miR-23a | AUCACAUUGCCAGGGAUUUCC | 66 |
| hsa-miR-23b | AUCACAUUGCCAGGGAUUACC | 67 |
| hsa-miR-24 | UGGCUCAGUUCAGCAGGAACAG | 68 |
| hsa-miR-25 | CAUUGCACUUGUCUCGGUCUGA | 69 |
| hsa-miR-27a | UUCACAGUGGCUAAGUUCCGC | 70 |
| hsa-miR-27b | UUCACAGUGGCUAAGUUCUGC | 71 |
| hsa-miR-28 | AAGGAGCUCACAGUCUAUUGAG | 72 |
| hsa-miR-296 | AGGGCCCCCCCUCAAUCCUGU | 73 |
| hsa-miR-299-3p | UAUGUGGGAUGGUAAACCGCUU | 74 |
| hsa-miR-299-5p | UGGUUUACCGUCCCACAUACAU | 75 |
| hsa-miR-29b | UAGCACCAUUUGAAAUCAGUGUU | 76 |
| hsa-miR-301 | CAGUGCAAUAGUAUUGUCAAAGC | 77 |
| hsa-miR-302a | UAAGUGCUUCCAUGUUUUGGUGA | 78 |
| hsa-miR-302b | UAAGUGCUUCCAUGUUUUAGUAG | 79 |
| hsa-miR-302c | UAAGUGCUUCCAUGUUUCAGUGG | 80 |
| hsa-miR-302c* | UUUAACAUGGGGGUACCUGCUG | 81 |
| hsa-miR-302d | UAAGUGCUUCCAUGUUUGAGUGU | 82 |
| hsa-miR-30a-3p | CUUUCAGUCGGAUGUUUGCAGC | 83 |
| hsa-miR-30a-5p | UGUAAACAUCCUCGACUGGAAG | 84 |
| hsa-miR-30b | UGUAAACAUCCUACACUCAGCU | 85 |
| hsa-miR-30c | UGUAAACAUCCUACACUCUCAGC | 86 |
| hsa-miR-30d | UGUAAACAUCCCCGACUGGAAG | 87 |
| hsa-miR-30e-3p | CUUUCAGUCGGAUGUUUACAGC | 88 |
| hsa-miR-324-5p | CGCAUCCCCUAGGGCAUUGGUGU | 89 |
| hsa-miR-325 | CCUAGUAGGUGUCCAGUAAGUGU | 90 |
| hsa-miR-326 | CCUCUGGGCCCUUCCUCCAG | 91 |
| hsa-miR-328 | CUGGCCCUCUCUGCCCUUCCGU | 92 |
| hsa-miR-329 | AACACACCUGGUUAACCUCUUU | 93 |
| hsa-miR-330 | GCAAAGCACACGGCCUGCAGAGA | 94 |
| hsa-miR-331 | GCCCCUGGGCCUAUCCUAGAA | 95 |
| hsa-miR-335 | UCAAGAGCAAUAACGAAAAAUGU | 96 |
| hsa-miR-346 | UGUCUGCCCGCAUGCCUGCCUCU | 97 |
| hsa-miR-34b | UAGGCAGUGUCAUUAGCUGAUUG | 98 |
| hsa-miR-34c | AGGCAGUGUAGUUAGCUGAUUGC | 99 |
| hsa-miR-361 | UUAUCAGAAUCUCCAGGGGUAC | 100 |
| hsa-miR-362 | AAUCCUUGGAACCUAGGUGUGAGU | 101 |
| hsa-miR-363 | AAUUGCACGGUAUCCAUCUGUA | 102 |
| hsa-miR-363* | CGGGUGGAUCACGAUGCAAUUU | 103 |
| hsa-miR-365 | UAAUGCCCCUAAAAAUCCUUAU | 104 |
| hsa-miR-367 | AAUUGCACUUUAGCAAUGGUGA | 105 |
| hsa-miR-369-3p | AAUAAUACAUGGUUGAUCUUU | 106 |
| hsa-miR-369-5p | AGAUCGACCGUGUUAUAUUCGC | 107 |
| hsa-miR-372 | AAAGUGCUGCGACAUUUGAGCGU | 108 |
| hsa-miR-373 | GAAGUGCUUCGAUUUUGGGGUGU | 109 |
| hsa-miR-373* | ACUCAAAAUGGGGGCGCUUUCC | 110 |
| hsa-miR-374 | UUAUAAUACAACCUGAUAAGUG | 111 |
| hsa-miR-375 | UUUGUUCGUUCGGCUCGCGUGA | 112 |
| hsa-miR-376a | AUCAUAGAGGAAAAUCCACGU | 113 |
| hsa-miR-376b | AUCAUAGAGGAAAAUCCAUGUU | 114 |
| hsa-miR-377 | AUCACACAAAGGCAACUUUUGU | 115 |
| hsa-miR-378 | CUCCUGACUCCAGGUCCUGUGU | 116 |
| hsa-miR-380-3p | UAUGUAAUAUGGUCCACAUCUU | 117 |
| hsa-miR-380-5p | UGGUUGACCAUAGAACAUGCGC | 118 |
| hsa-miR-381 | UAUACAAGGGCAAGCUCUCUGU | 119 |
| hsa-miR-382 | GAAGUUGUUCGUGGUGGAUUCG | 120 |
| hsa-miR-383 | AGAUCAGAAGGUGAUUGUGGCU | 121 |
| hsa-miR-384 | AUUCCUAGAAAUUGUUCAUA | 122 |
| hsa-miR-410 | AAUAUAACACAGAUGGCCUGU | 123 |
| hsa-miR-411 | UAGUAGACCGUAUAGCGUACG | 124 |
| hsa-miR-412 | ACUUCACCUGGUCCACUAGCCGU | 125 |
| hsa-miR-421 | AUCAACAGACAUUAAUUGGGCGC | 126 |
| hsa-miR-422a | CUGGACUUAGGGUCAGAAGGCC | 127 |
| hsa-miR-422b | CUGGACUUGGAGUCAGAAGGCC | 128 |
| hsa-miR-424 | CAGCAGCAAUUCAUGUUUUGAA | 129 |
| hsa-miR-425-5p | AAUGACACGAUCACUCCCGUUGA | 130 |
| hsa-miR-429 | UAAUACUGUCUGGUAAAACCGU | 131 |
| hsa-miR-431 | UGUCUUGCAGGCCGUCAUGCA | 132 |
| hsa-miR-432 | UCUUGGAGUAGGUCAUUGGGUGG | 133 |
| hsa-miR-432* | CUGGAUGGCUCCUCCAUGUCU | 134 |
| hsa-miR-433 | AUCAUGAUGGGCUCCUCGGUGU | 135 |
| hsa-miR-448 | UUGCAUAUGUAGGAUGUCCCAU | 136 |
| hsa-miR-449 | UGGCAGUGUAUUGUUAGCUGGU | 137 |
| hsa-miR-449b | AGGCAGUGUAUUGUUAGCUGGC | 138 |
| hsa-miR-454-5p | ACCCUAUCAAUAUUGUCUCUGC | 139 |
| hsa-miR-455 | UAUGUGCCUUUGGACUACAUCG | 140 |
| hsa-miR-484 | UCAGGCUCAGUCCCCUCCCGAU | 141 |
| hsa-miR-485-3p | GUCAUACACGGCUCUCCUCUCU | 142 |
| hsa-miR-485-5p | AGAGGCUGGCCGUGAUGAAUUC | 143 |
| hsa-miR-486 | UCCUGUACUGAGCUGCCCCGAG | 144 |
| hsa-miR-487a | AAUCAUACAGGGACAUCCAGUU | 145 |
| hsa-miR-487b | AAUCGUACAGGGUCAUCCACUU | 146 |
| hsa-miR-488 | CCCAGAUAAUGGCACUCUCAA | 147 |
| hsa-miR-490 | CAACCUGGAGGACUCCAUGCUG | 148 |
| hsa-miR-492 | AGGACCUGCGGGACAAGAUUCUU | 149 |
| hsa-miR-493-5p | UUGUACAUGGUAGGCUUUCAUU | 150 |
| hsa-miR-497 | CAGCAGCACACUGUGGUUUGU | 151 |
| hsa-miR-498 | UUUCAAGCCAGGGGGCGUUUUUC | 152 |
| hsa-miR-500 | AUGCACCUGGGCAAGGAUUCUG | 153 |
| hsa-miR-501 | AAUCCUUUGUCCCUGGGUGAGA | 154 |
| hsa-miR-502 | AUCCUUGCUAUCUGGGUGCUA | 155 |
| hsa-miR-503 | UAGCAGCGGGAACAGUUCUGCAG | 156 |
| hsa-miR-506 | UAAGGCACCCUUCUGAGUAGA | 157 |
| hsa-miR-507 | UUUUGCACCUUUUGGAGUGAA | 158 |
| hsa-miR-508 | UGAUUGUAGCCUUUUGGAGUAGA | 159 |
| hsa-miR-511 | GUGUCUUUUGCUCUGCAGUCA | 160 |
| hsa-miR-512-3p | AAGUGCUGUCAUAGCUGAGGUC | 161 |
| hsa-miR-512-5p | CACUCAGCCUUGAGGGCACUUUC | 162 |
| hsa-miR-515-5p | UUCUCCAAAAGAAAGCACUUUCUG | 163 |
| hsa-miR-516-3p | UGCUUCCUUUCAGAGGGU | 164 |
| hsa-miR-517* | CCUCUAGAUGGAAGCACUGUCU | 165 |
| hsa-miR-517b | UCGUGCAUCCCUUUAGAGUGUU | 166 |
| hsa-miR-517c | AUCGUGCAUCCUUUUAGAGUGU | 167 |
| hsa-miR-518b | CAAAGCGCUCCCCUUUAGAGGU | 168 |
| hsa-miR-518c* | UCUCUGGAGGGAAGCACUUUCUG | 169 |
| hsa-miR-518d | CAAAGCGCUUCCCUUUGGAGC | 170 |
| hsa-miR-519c | AAAGUGCAUCUUUUUAGAGGAU | 171 |
| hsa-miR-519e* | UUCUCCAAAAGGGAGCACUUUC | 172 |
| hsa-miR-520a | AAAGUGCUUCCCUUUGGACUGU | 173 |
| hsa-miR-520a* | CUCCAGAGGGAAGUACUUUCU | 174 |
| hsa-miR-520e | AAAGUGCUUCCUUUUUGAGGG | 175 |
| hsa-miR-520f | AAGUGCUUCCUUUUAGAGGGUU | 176 |
| hsa-miR-520g | ACAAAGUGCUUCCCUUUAGAGUGU | 177 |
| hsa-miR-520h | ACAAAGUGCUUCCCUUUAGAGU | 178 |
| hsa-miR-521 | AACGCACUUCCCUUUAGAGUGU | 179 |
| hsa-miR-524 | GAAGGCGCUUCCCUUUGGAGU | 180 |
| hsa-miR-524* | CUACAAAGGGAAGCACUUUCUC | 181 |
| hsa-miR-525 | CUCCAGAGGGAUGCACUUUCU | 182 |
| hsa-miR-526b* | AAAGUGCUUCCUUUUAGAGGC | 183 |
| hsa-miR-532 | CAUGCCUUGAGUGUAGGACCGU | 184 |
| hsa-miR-539 | GGAGAAAUUAUCCUUGGUGUGU | 185 |
| hsa-miR-542-3p | UGUGACAGAUUGAUAACUGAAA | 186 |
| hsa-miR-548a | CAAAACUGGCAAUUACUUUUGC | 187 |
| hsa-miR-548b | CAAGAACCUCAGUUGCUUUUGU | 188 |
| hsa-miR-548c | CAAAAAUCUCAAUUACUUUUGC | 189 |
| hsa-miR-548d | CAAAAACCACAGUUUCUUUUGC | 190 |
| hsa-miR-549 | UGACAACUAUGGAUGAGCUCU | 191 |
| hsa-miR-550 | UGUCUUACUCCCUCAGGCACAU | 192 |
| hsa-miR-551a | GCGACCCACUCUUGGUUUCCA | 193 |
| hsa-miR-551b | GCGACCCAUACUUGGUUUCAG | 194 |
| hsa-miR-552 | AACAGGUGACUGGUUAGACAA | 195 |
| hsa-miR-553 | AAAACGGUGAGAUUUUGUUUU | 196 |
| hsa-miR-554 | GCUAGUCCUGACUCAGCCAGU | 197 |
| hsa-miR-555 | AGGGUAAGCUGAACCUCUGAU | 198 |
| hsa-miR-557 | GUUUGCACGGGUGGGCCUUGUCU | 199 |
| hsa-miR-558 | UGAGCUGCUGUACCAAAAU | 200 |
| hsa-miR-559 | UAAAGUAAAUAUGCACCAAAA | 201 |
| hsa-miR-560 | GCGUGCGCCGGCCGGCCGCC | 202 |
| hsa-miR-561 | CAAAGUUUAAGAUCCUUGAAGU | 203 |
| hsa-miR-562 | AAAGUAGCUGUACCAUUUGC | 204 |
| hsa-miR-563 | AGGUUGACAUACGUUUCCC | 205 |
| hsa-miR-564 | AGGCACGGUGUCAGCAGGC | 206 |
| hsa-miR-565 | GGCUGGCUCGCGAUGUCUGUUU | 207 |
| hsa-miR-566 | GGGCGCCUGUGAUCCCAAC | 208 |
| hsa-miR-567 | AGUAUGUUCUUCCAGGACAGAAC | 209 |
| hsa-miR-568 | AUGUAUAAAUGUAUACACAC | 210 |
| hsa-miR-569 | AGUUAAUGAAUCCUGGAAAGU | 211 |
| hsa-miR-571 | UGAGUUGGCCAUCUGAGUGAG | 212 |
| hsa-miR-572 | GUCCGCUCGGCGGUGGCCCA | 213 |
| hsa-miR-573 | CUGAAGUGAUGUGUAACUGAUCAG | 214 |
| hsa-miR-575 | GAGCCAGUUGGACAGGAGC | 215 |
| hsa-miR-577 | UAGAUAAAAUAUUGGUACCUG | 216 |
| hsa-miR-578 | CUUCUUGUGCUCUAGGAUUGU | 217 |
| hsa-miR-580 | UUGAGAAUGAUGAAUCAUUAGG | 218 |
| hsa-miR-581 | UCUUGUGUUCUCUAGAUCAGU | 219 |
| hsa-miR-582 | UUACAGUUGUUCAACCAGUUACU | 220 |
| hsa-miR-583 | CAAAGAGGAAGGUCCCAUUAC | 221 |
| hsa-miR-584 | UUAUGGUUUGCCUGGGACUGAG | 222 |
| hsa-miR-585 | UGGGCGUAUCUGUAUGCUA | 223 |
| hsa-miR-586 | UAUGCAUUGUAUUUUUAGGUCC | 224 |
| hsa-miR-587 | UUUCCAUAGGUGAUGAGUCAC | 225 |
| hsa-miR-588 | UUGGCCACAAUGGGUUAGAAC | 226 |
| hsa-miR-589 | UCAGAACAAAUGCCGGUUCCCAGA | 227 |
| hsa-miR-590 | GAGCUUAUUCAUAAAAGUGCAG | 228 |
| hsa-miR-591 | AGACCAUGGGUUCUCAUUGU | 229 |
| hsa-miR-592 | UUGUGUCAAUAUGCGAUGAUGU | 230 |
| hsa-miR-593 | AGGCACCAGCCAGGCAUUGCUCAGC | 231 |
| hsa-miR-595 | GAAGUGUGCCGUGGUGUGUCU | 232 |
| hsa-miR-596 | AAGCCUGCCCGGCUCCUCGGG | 233 |
| hsa-miR-597 | UGUGUCACUCGAUGACCACUGU | 234 |
| hsa-miR-598 | UACGUCAUCGUUGUCAUCGUCA | 235 |
| hsa-miR-599 | GUUGUGUCAGUUUAUCAAAC | 236 |
| hsa-miR-600 | ACUUACAGACAAGAGCCUUGCUC | 237 |
| hsa-miR-601 | UGGUCUAGGAUUGUUGGAGGAG | 238 |
| hsa-miR-602 | GACACGGGCGACAGCUGCGGCCC | 239 |
| hsa-miR-603 | CACACACUGCAAUUACUUUUGC | 240 |
| hsa-miR-604 | AGGCUGCGGAAUUCAGGAC | 241 |
| hsa-miR-605 | UAAAUCCCAUGGUGCCUUCUCCU | 242 |
| hsa-miR-606 | AAACUACUGAAAAUCAAAGAU | 243 |
| hsa-miR-607 | GUUCAAAUCCAGAUCUAUAAC | 244 |
| hsa-miR-608 | AGGGGUGGUGUUGGGACAGCUCCGU | 245 |
| hsa-miR-609 | AGGGUGUUUCUCUCAUCUCU | 246 |
| hsa-miR-610 | UGAGCUAAAUGUGUGCUGGGA | 247 |
| hsa-miR-611 | GCGAGGACCCCUCGGGGUCUGAC | 248 |
| hsa-miR-612 | GCUGGGCAGGGCUUCUGAGCUCCUU | 249 |
| hsa-miR-613 | AGGAAUGUUCCUUCUUUGCC | 250 |
| hsa-miR-614 | GAACGCCUGUUCUUGCCAGGUGG | 251 |
| hsa-miR-616 | ACUCAAAACCCUUCAGUGACUU | 252 |
| hsa-miR-617 | AGACUUCCCAUUUGAAGGUGGC | 253 |
| hsa-miR-618 | AAACUCUACUUGUCCUUCUGAGU | 254 |
| hsa-miR-619 | GACCUGGACAUGUUUGUGCCCAGU | 255 |
| hsa-miR-620 | AUGGAGAUAGAUAUAGAAAU | 256 |
| hsa-miR-621 | GGCUAGCAACAGCGCUUACCU | 257 |
| hsa-miR-622 | ACAGUCUGCUGAGGUUGGAGC | 258 |
| hsa-miR-623 | AUCCCUUGCAGGGGCUGUUGGGU | 259 |
| hsa-miR-624 | UAGUACCAGUACCUUGUGUUCA | 260 |
| hsa-miR-626 | AGCUGUCUGAAAAUGUCUU | 261 |
| hsa-miR-627 | GUGAGUCUCUAAGAAAAGAGGA | 262 |
| hsa-miR-629 | GUUCUCCCAACGUAAGCCCAGC | 263 |
| hsa-miR-630 | AGUAUUCUGUACCAGGGAAGGU | 264 |
| hsa-miR-631 | AGACCUGGCCCAGACCUCAGC | 265 |
| hsa-miR-632 | GUGUCUGCUUCCUGUGGGA | 266 |
| hsa-miR-633 | CUAAUAGUAUCUACCACAAUAAA | 267 |
| hsa-miR-634 | AACCAGCACCCCAACUUUGGAC | 268 |
| hsa-miR-635 | ACUUGGGCACUGAAACAAUGUCC | 269 |
| hsa-miR-637 | ACUGGGGGCUUUCGGGCUCUGCGU | 270 |
| hsa-miR-638 | AGGGAUCGCGGGCGGGUGGCGGCCU | 271 |
| hsa-miR-639 | AUCGCUGCGGUUGCGAGCGCUGU | 272 |
| hsa-miR-640 | AUGAUCCAGGAACCUGCCUCU | 273 |
| hsa-miR-641 | AAAGACAUAGGAUAGAGUCACCUC | 274 |
| hsa-miR-642 | GUCCCUCUCCAAAUGUGUCUUG | 275 |
| hsa-miR-643 | ACUUGUAUGCUAGCUCAGGUAG | 276 |
| hsa-miR-644 | AGUGUGGCUUUCUUAGAGC | 277 |
| hsa-miR-645 | UCUAGGCUGGUACUGCUGA | 278 |
| hsa-miR-646 | AAGCAGCUGCCUCUGAGGC | 279 |
| hsa-miR-647 | GUGGCUGCACUCACUUCCUUC | 280 |
| hsa-miR-648 | AAGUGUGCAGGGCACUGGU | 281 |
| hsa-miR-649 | AAACCUGUGUUGUUCAAGAGUC | 282 |
| hsa-miR-650 | AGGAGGCAGCGCUCUCAGGAC | 283 |
| hsa-miR-651 | UUUAGGAUAAGCUUGACUUUUG | 284 |
| hsa-miR-654 | UGGUGGGCCGCAGAACAUGUGC | 285 |
| hsa-miR-655 | AUAAUACAUGGUUAACCUCUUU | 286 |
| hsa-miR-656 | AAUAUUAUACAGUCAACCUCU | 287 |
| hsa-miR-657 | GGCAGGUUCUCACCCUCUCUAGG | 288 |
| hsa-miR-658 | GGCGGAGGGAAGUAGGUCCGUUGGU | 289 |
| hsa-miR-659 | CUUGGUUCAGGGAGGGUCCCCA | 290 |
| hsa-miR-660 | UACCCAUUGCAUAUCGGAGUUG | 291 |
| hsa-miR-661 | UGCCUGGGUCUCUGGCCUGCGCGU | 292 |
| hsa-miR-662 | UCCCACGUUGUGGCCCAGCAG | 293 |
| hsa-miR-663 | AGGCGGGGCGCCGCGGGACCGC | 294 |
| hsa-miR-668 | UGUCACUCGGCUCGGCCCACUAC | 295 |
| hsa-miR-758 | UUUGUGACCUGGUCCACUAACC | 296 |
| hsa-miR-765 | UGGAGGAGAAGGAAGGUGAUG | 297 |
| hsa-miR-766 | ACUCCAGCCCCACAGCCUCAGC | 298 |
| hsa-miR-767-3p | UCUGCUCAUACCCCAUGGUUUCU | 299 |
| hsa-miR-767-5p | UGCACCAUGGUUGUCUGAGCAUG | 300 |
| hsa-miR-768-3p | UCACAAUGCUGACACUCAAACUGCUGAC | 301 |
| hsa-miR-768-5p | GUUGGAGGAUGAAAGUACGGAGUGAU | 302 |
| hsa-miR-769-3p | CUGGGAUCUCCGGGGUCUUGGUU | 303 |
| hsa-miR-769-5p | UGAGACCUCUGGGUUCUGAGCU | 304 |
| hsa-miR-770-5p | UCCAGUACCACGUGUCAGGGCCA | 305 |
| hsa-miR-801 | GAUUGCUCUGCGUGCGGAAUCGAC | 306 |
| hsa-miR-802 | CAGUAACAAAGAUUCAUCCUUGU | 307 |
| hsa-miR-9 | UCUUUGGUUAUCUAGCUGUAUGA | 308 |
| hsa-miR-95 | UUCAACGGGUAUUUAUUGAGCA | 309 |
| hsa-miR-98 | UGAGGUAGUAAGUUGUAUUGUU | 310 |
| hsa-miR-99a | AACCCGUAGAUCCGAUCUUGUG | 311 |
| hsa-miR-99b | CACCCGUAGAACCGACCUUGCG | 312 |
| hsv-1miR-LAT | UGGCGGCCCGGCCCGGGGCC | 313 |

In a most preferred embodiment, the seed sequence of the micro RNA is position 1-7of any SEQ ID NOs:1-313.

### Activity of the oligonucleotide of the invention

### RNase H cleavage

RNase H will cleave the RNA part of a RNA-DNA duplex. The structural requirements for RNase H activation are well-known to the skilled man. This mechanism is very often used to achieve traditional antisense regulation e.g. by employing so-called gapmers. Gapmers are antisense oligonucleotides that comprise a central region with deoxy sugars (the gap) and modified flanks. Gapmers very often comprises phosphorothioate internucleotide linkages to improve biostability and the flanks comprise e.g. 2-O-modifications that also improve biostability, i.e. resistance against nucleolytic attack. The flanks may also comprise modifications that increase the melting temperature of the gapmer base paired to a complementary nucleic acid. Also headmer and endmer structures have been described in the literature.

The oligonucleotide of the invention is not capable of inducing RNase H cleavage of the target RNA. The skilled man is well aware of the requirements for RNase H cleavage and will be able to design oligonucleotides that do or do not activate RNase H.

Thus, in a preferred embodiment, the oligonucleotide does not comprise a stretch of unmodified DNA that exceeds a length selected from the group consisting of: 3 bases, 4 bases, 5 bases, 6 bases, 7 bases, 8 bases, 9 bases, 10 bases and 11 bases. Most preferably, the stretch of unmodified DNA does not exceed 3 bases.

In another preferred embodiment, the oligonucleotide does not comprise any DNA monomers.

### Recruiting the RNAi machinery

The RNAi machinery is a sophisticated gene regulatory system that is guided by RNA. Thus, microRNAs guide the RNAi machinery to target mRNAs to affect the activity of the target mRNA. The RNAi machinery may affect translation of the mRNA directly or it may affect the stability of the target mRNA, i.e. mediate direct degradation of the target mRNA. Not intended to be bound by theory, it is believed that the degree of complementarity between microRNA and target mRNA is a key element as to whether the target mRNA is subjected to translational regulation or degradation.

Endogenous microRNAs are processed from precursor stem-loops and incorporated into a so called RNA induced silencing complex (RISC complex). The details of this process are still poorly understood.

The cellular RNAi machinery has been extensively used to affect the activity of cellular mRNAs by introducing synthetic double stranded RNA complexes termed siRNAs into the cell. As mentioned above, siRNAs are short double stranded RNA complexes comprising a passenger strand and a complementary guide strand. The guide strand of siRNA is incorporated into the RISC complex, where after the RISC complex can affect the activity of mRNA harbouring complementary sequences to the guide strand. Thus, siRNAs are a new class of compounds that is thought to be capable of efficiently and specifically targeting any mRNA and consequently, siRNAs are regarded potentially as a new class of therapeutics.

A common feature of siRNAs and microRNAs is that they recruit the cellular RNAi complex to affect the activity of target RNAs.

### Blockmir

The oligonucleotides cannot recruit the RNAi machinery. and it is preferred that the oligonucleotides of the invention are capable of blocking the activity of the RNAi machinery at a particular target RNA. As mentioned above, the oligonucleotides may do so by sequestering the target sequence of the target RNA, such that the RNAi machinery will not recognize the target sequence, as it is base paired to the oligonucleotides. Oligonucleotides of the invention with this activity may also be referred to as blockmirs.

In a preferred embodiment, the oligonucleotide is capable of blocking the regulatory activity of a microRNA at a particular target RNA. Preferably, the microRNA is an endogenous microRNA.

After the priority date of this patent application, examples of oligonucleotides capable of blocking the regulatory activity of a microRNA at a given mRNA has been published by two groups.

In the first publication (Xiao J, 2007), oligonucleotides termed microRNA masking antisense ODN (oligodeoxynucleotides) was used to interfere with the regulatory activity of mir-1 on HCN2 and HCN4 and the regulatory activity of mir-133 on HCN2. It was observed that microRNA masking antisense increased the protein level of HCN2 and HCN4 in a gene specific manner, as determined by immunoblotting using cultured neonatal rat ventricular cells and luciferase assays using HEK293 human embryonic kidney cell line. I.e. the mechanism of action of blockmirs was validated. In other words, it was demonstrated that an oligonucleotide that binds to the target site of a microRNA in the 3'UTR of a mRNA, can prevent microRNA regulation of the mRNA in mammalian cells (rat and human).

However, the design of the blockmirs in the work of Xiao et al., 2007 left some questions open. The microRNA masking antisense ODN consisted of deoxynucleotides with 5 LNA monomers at both ends. Thus, the central part of the oligonucleotide apparently consisted of a stretch of 12 unmodified deoxynucleotides. Such structure is typically expected to activate RNase H and hence mediate degradation of target RNAs.

In the second publication (Choi WY, 2007) blockmirs (termed target protectors) was used to prevent microRNA regulation of specific mRNAs in zebrafish. More specifically, the authors used morpholino oligonucleotides of 25 units with perfect complementarity to zebrafish mir-430 target sites in *squint* and *lefty* mRNA to prevent mir-430 regulation of the target mRNAs (*squint* and *lefty*). Thus, the authors validate the blockmir approach in a different organism than did Xiao et al., and they also validate that a different chemistry can be used.

We suggest that the essence of blockmir activity is binding to a microRNA target site, and that this can achieved using a variety of chemistries and also in a variety of organisms.

Another report published after the priority date of this patent application studied the molecular basis for target RNA recognition and cleavage by human RISC (Ameres SL, 2007). These authors found that target accessibility determines RISC mediated cleavage in vitro and in vivo. Among others, they blocked target accessibility using oligonucleotides complementary to a siRNA target site, i.e. the oligonucleotides may be seen as functional analogues of the blockmirs of the present invention, except that they target a siRNA target site that is regulated by a siRNA with perfect complementary. Interestingly, the authors found that blocking 3 or 6 nt of the 21 nt target sequence in the region annealing to the 3'part of the siRNA had no effect on regulation (as seen by cleavage rates using affinity purified human RISC). In contrast, blocking 5 nt of the target site in the region annealing to the 5'part of the siRNA severely impaired regulation (as seen by cleavage) and even blocking only 2 nt impaired regulation.

Returning to blockmirs of the invention, if the microRNA is a positive regulator of the target RNA, the oligonucleotide will be a negative regulator of the target RNA.

Most often, the microRNA is a negative regulator of the target RNA. Thus, in another embodiment, the oligonucleotide is a positive regulator of the target RNA. This is contrary to traditional antisense oligonucleotides, microRNAs and siRNAs that typically act as negative regulators.

In a preferred embodiment, the blockmirs of the invention are DNAs, as these will not be recognized by the RNAi machinery and consequently function as neither microRNA nor siRNA. Preferably, the DNA units are modified such as to prevent RNase H activation. Alternatively, less than 5 consecutive DNA units are present, such as less than 4 consecutive DNA and less than 3 consecutive DNA units.

In still another embodiment, the blockmir does not comprise any DNA units.

In yet another embodiment, the blockmir does not comprise any RNA units.

In another embodiment, the blockmir does not comprise a stretch of RNA units that exceeds a length selected from the group of consisting of: a length of 5 units, 6 units, 7 units, 8 units, 9 units, 10 units, 11 units, 12 units, 13 units, 14 units, 15 units, 16 units, 17 units, 18 units, 19 units, 20 units, 21 units and 22 units.

In one embodiment, the oligonucleotides have been chemically modified such as to not being capable of recruiting the RNAi machinery. Preferred modifications include 2'-O-modications such as 2'-O-methyl and 2'O-F. Also conjugated RNAs are preferred. E.g. RNAs conjugated to a cholesterol moiety, in which case the cholesterol may both prevent the oligonucleotide from recruiting the RNAi machinery and improve the bioavailability of the oligonucleotide. The cholesterol moiety may be conjugated to a monomer within the guide sequence of the oligonucleotide or at the 3'end or the 5'end of the oligonucleotide. More modifications are described below.

In yet another embodiment, the blockmir may comprise a mix of DNA units and RNA units such as to prevent the oligonucleotide from activating RNase H and to at the same time prevent the oligonucleotide from recruiting the RNAi machinery. E.g. a DNA unit may be followed by a RNA unit that is again followed by a DNA unit and so on. Further, in a preferred embodiment, phosphorothioate internucleotide linkages may connect the units to improve the biostability of the oligonucleotide. Both DNA units and RNA units may be modified. Preferably, RNA units are modified in the 2'-O-position (2'-O-methyl, LNA etc.).

In yet another embodiment, the oligonucleotide (blockmir) comprise a mix of DNA units and RNA units such as to prevent the oligonucleotide from activating RNase H and to at the same time prevent the oligonucleotide from recruiting the RNAi machinery, wherein the DNA units and RNA units come in blocks. The blocks may have a length of 2 units, 3 units, 4 units, 5 units or 6 units and units of different length may be comprised with the same oligonucleotide. Both DNA units and RNA units may be modified. Preferably, RNA units are modified in the 2'-O-position (2'-O-methyl, LNA etc).

In a preferred embodiment, also units selected from the group of LNA units, INA units and morpholino units are comprised within the oligonucleotide. In another preferred embodiment, the oligonucleotide comprises a mix of LNA units and RNA units with a 2'-O-methyl. Such mixmers have been used as steric block inhibitors of Human Immunodeficiency Virus Type 1 Tat-Dependent Trans-Activation and HIV-1 Infectivity.

In still another embodiment, the blockmir are entirely composed of units selected from the group of 2'-O-methyl modified units, LNA units, PNA units, INA units and morpholino units. In one embodiment, the units are mixed, while in another embodiment, the blockmir is composed of only one of the units.

In still another embodiment, the blockmir has been designed such as to able to bind to more than one target RNA. Promiscuity may be designed into blockmirs using universal bases. Also reducing the length of the blockmir will increase promiscuity. Thus, in one embodiment, the blockmir may only consist of the guide sequence corresponding to a seed sequence of a microRNA. In this embodiment, it is preferred that affinity increasing modifications are are used and the oligonucleotide may be fully modified in the 2'O-position with e.g. 2'-O-methyl, 2'-O-'flouro, 2'-O-(2-methoxyethyl) or the nucleotides may be locked (LNA).

### Off-target effects

In most embodiments, off-target binding of the blockmir will have very few or no effects. This is contrary to antimirs, RNAi mediated by siRNAs and microRNAs, and RNase H mediated antisense regulation, which may all give rise to off-targets effects. The blockmir only has an effect if it binds to a microRNA target region and thereby prevents microRNA regulation of the target RNA.

Thus, in a preferred embodiment, the blockmir will have reduced off-target effects, as compared to regulating the activity of the target mRNA using an antimir.

An antimir, as used in the present context, is an oligonucleotide that can base pair with a microRNA and thereby inhibit the activity of the microRNA. Since most microRNAs are promiscuous, i.e. they regulate more than one target, regulation of a particular microRNA will affect the activity of more than one target mRNA. Thus, when it is desired to only regulate the activity of one particular target mRNA, regulation of other target mRNAs may be referred to as off-target effects of the antimir.

Using a (exogenous) promiscuous microRNA to affect or regulate the activity of a target mRNA, instead of an antimir may obviously also have off-target effects.

Moreover, the target repertoire of a given microRNA may vary in different cells, wherefore an antimir may have different off target effects in different cells. Likewise for regulation using a promiscuous microRNA. A blockmir will only have an effect in the particular cells wherein the target RNA is regulated by a microRNA. Thus, a blockmir enables targeting of cell specific microRNA:mRNA interactions. If the blockmir enter a cell that does not have the particular microRNA:mRNA interaction, the blockmir will have little or no effect.

siRNAs are double stranded RNA complexes comprising a passenger strand and a guide strand that mediate degradation of target mRNAs that are complementary to the guide strand of the RNA complex. It has now been recognized that siRNAs often have off-target effects, because the strand acting as guide strand can also function as microRNA, i.e. siRNAs may mediate regulation of target mRNAs that are not fully complementary to the guide strand of the siRNA.

Thus, in one embodiment, an blockmir of the present invention will have reduced off-target effects as compared to a siRNA directed to the same target mRNA.

In another preferred embodiment, the blockmir will also have reduced off-target effects as compared to using a traditional antisense oligonucleotide for regulation of the target mRNA.

Traditional antisense oligonucleotides are often designed such as to mediate RNase H cleavage of their target RNA. RNase H cleaves a duplex of RNA and DNA. Thus, if such an antisense oligonucleotide base pairs to a non-intended mRNA, this mRNA will be inactivated by RNase H cleavage, and hence giving rise to off-target effects.

In conclusion, blockmirs of the present invention are characteristic in that they affect the activity of an RNA by preventing microRNA regulation of the target RNA. Thus, blockmirs of the present invention will have reduced off target effects as compared to both traditional antisense oligonucleotides, antimirs, and RNAi mediated regulation using microRNAs and siRNAs.

A consideration when designing short blockmirs is obviously that the transcriptome may comprise more than one site with perfect complementary to the blockmir. However, as outlined above, the blockmirs will only affect the target RNA if the target sequence is also a target sequence for microRNA regulation. Therefore, even very short blockmirs may have very little of no off-target effects. Thus, the blockmirs may deliberately be designed to target many sites. The blockmirs will then preferentially bind to microRNA target sites since these are more accessible, and the blockmirs will only have effects if they prevent microRNA binding to a target site.

### Chemistry

The oligonucleotides of the invention comprises nucleotide monomers that increase its affinity for complementary sequences or affinity increasing modifications. This is particular relevant for short oligonucleotides and may allow for generation of very short active oligonucleotides, e.g. of a length between 10 and 15 bases or even less than 10 bases, such as e.g. only the guide sequence corresponding to the seed sequence of a microRNA.

Nucleotide units that increase the affinity for complementary sequences may e.g. be LNA (locked nucleic acid) units, PNA (peptide nucleic acid) units or INA (intercalating nucleic acid) units. Also RNA units modified in the 2-O-position (e.g. 2'-O-(2-methoxyethyl)-RNA, 2'O-methyl-RNA, 2'O-flouro-RNA) increase the affinity for complementary sequences. At the same time, such modifications often also improve the biostability of the oligonucleotides, as they become a poorer substrate for cellular nucleases.

The oligonucleotide may also comprise modifications that increase its biostability and/or bioavailability, such as phosphorothioate linkages. The oligonucleotide may be fully phosphorothiolated or only partly phosphorothiolated.

In a preferred embodiment, the oligonucleotide comprises a repeating pattern of one or more LNA units and one or more units that are substituted in the 2'-position. OMe/LNA mixmers have been shown to be powerful reagents for use as steric block inhibitors of gene expression regulated by protein-RNA interactions. Thus, when the oligonucleotides of the invention are used to block the activity of a microRNA at a target RNA, a OMe/LNA mixmer architecture may be used. A gapmer structure structure may also be used, however preferably without being capable of inducing RNase H if the oligonucleotide is intended to act as a blockmir.

In one embodiment, the oligonucleotide of the invention does not comprise any RNA units. Few or no RNA units may be used to prevent the oligonucleotide from being capable of recruiting the RNAi machinery. Chemical modifications can do the same.

In another embodiment, the oligonucleotide of the invention does not comprise any DNA units.

In still another embodiment, the oligonucleotide of the invention does not comprise any morpholino units and/or LNA units.

In yet another embodiment, the oligonucleotide comprises modifications that increase its biostability. The modifications may be the nucleotide units mentioned above for increasing the affinity toward complementary sequences.

In a preferred embodiment, the oligonucleotides comprise a number of nucleotide units that increase the affinity for complementary sequences selected from the group of: 1 units, 2 units, 3 units, 4 units, 5 units, 6 units, 7 units, 8 units, 9 units, 10 units, 11 units, 12 units, 13 units, 14 units, 15 units, 16 units, 17 units, 18 units, 19 units, 20 units, 21 units, and 22 units.

In a preferred embodiment, nucleotide units that increase the affinity for complementary sequences are located at the flanks of the oligonucleotide. E.g. if the oligonucleotide comprise e.g. 10 LNA units, 5 may be located at the 5'end and the other 5 units may be located at the 3'end.

In still another embodiment, the oligonucleotides comprise modifications that increase its bioavailability. Modifications that improve cellular delivery are particular preferred.

### Promiscuity and specificity

In yet another embodiment, the oligonucleotide of the present invention may comprise nucleotides that do not hybridise specifically. Such nucleotides comprise so called universal bases. These are characterised in that they fit into a Watson-crick helix opposite to any base. Thus, they may be used to impose a certain degree of promiscuity on the oligonucleotides of the invention. That may e.g. be employed if the oligonucleotide is intended to target two particular mRNAs.

In a preferred embodiment, it may be desired to target most or all targets of a particular microRNA. In such case, the oligonucleotide may comprise a guide sequence corresponding to the seed sequence of the microRNA and one or two blocks of natural bases. The size of the blocks of natural bases can be adjusted such as to achieve a reasonable affinity to target sequences.

In still another embodiment, the oligonucleotide of the invention comprises a universal base selected from the group consisting of 3-nitropyrrole, 5-nitroindole, 3-methyl isocarbostyril or 5-methyl isocarbostyril.

In one embodiment, the oligonucleotides of the invention may comprise a guide sequence which is flanked by universal bases on the 3'side, the 5'side or both. Such an oligonucleotide may be used to mimic the promiscuous specificity of a microRNA and hence, block the activity of the microRNA at multiple target RNAs or even all target RNAs of the microRNAs. A combination of universal bases and e.g. inosine may also be used to design an oligonucleotide that only targets a subset of the target RNAs of a microRNA.

Universal bases tend to decrease the melting temperature of the oligonucleotide, wherefore it is preferred to counteract this decrease by incorporation of affinity increasing modifications or units, e.g. LNA units or 2'-O-methyl groups.

### Delivery

Various methods for delivery of oligonucleotides are known to the skilled man. Thus, oligonucleotides may be formulated in microparticles and nanoparticles. Liposomes are frequently used as delivery vehicle and a variety of liposome delivery systems exist. They may e.g. comprise cationic lipids or neutral lipids. Their size may be varied for various purposes and other components may be included in the liposomes or on the surface of the liposomes. Chitosan nanoparticles have been used for delivery of plasmids and siRNAs to various cells, among them primary cells. Thus, chitosan nanoparticles may also be used for delivery of the oligonucleotides of the invention. Others polymers for delivery are polyethyleneimine (PEI), cyclodextrin, atelocollagen, polyamidoamine (PAMAM) and poly(lactic-co-glycolic acid) (PLGA). Further, oligonucleotides of the invention may be conjugated to cationic peptides that have been shown to facilitate transport into cells.

### -Ex vivo or in vitro method of modulating the activity of a target RNA

A second aspect of the invention is an ex vivo or in vitro method of modulating the activity of a target mRNA by preventing the activity of a microRNA at the target mRNA comprising the steps of:
a. Providing a system comprising a target mRNA
b. Providing an oligonucleotide
c. Introducing the oligonucleotide of step b to the system of step a
d. Thereby modulating the activity of the target mRNA
   - wherein the antisense sequence of the oligonucleotide comprises a continuous stretch of less than 35 bases and more than 7 bases that is complementary to the target sequence, said target sequence being the sequence of the target mRNA involved in microRNA regulation and
      i. wherein said target sequence comprises an anti-seed sequence complementary to the seed sequence of the microRNA regulating the target RNA,
      ii. the seed sequence is position 2-7 counted from the 5'end of the microRNA.
      iii. wherein said continuous stretch of bases comprise a guide sequence that can base pair to the anti-seed of the target sequence and,
      iv. the target sequence of the target mRNA resides within the 3'UTR of the target mRNA
   - the oligonucleotide is not capable of inducing RNase H cleavage and not capable of recruiting the RNAi machinery, and
   - the oligonucleotide that has a degree of identity to the microRNA of less than 90%, wherein the degree of identity is counted over the length of the shortest molecule of the microRNA and the oligonucleotide, and
   - the oligonucleotide has reduced off-target effects, as compared to regulating the activity of the target mRNA using an oligonucleotide that can base pair with a microRNA and thereby inhibit the activity of the microRNA and
   - the oligonucleotide comprises nucleotide monomers that increase its affinity for complementary sequences or affinity increasing modifications.
   - wherein the sequence of the microRNA is selected from the group consisting of: SEQ ID NOs: 1-313.

Preferably, the system is either a cell extract or a cell.
In one embodiment, the method is a method for validating the activity of the oligonucleotide, i.e. verifying whether the oligonucleotide can indeed modulate the activity of the target RNA and to what extent. Such method may be used when aiming to identify oligonucleotides with optimal activity e.g. for therapeutic development. In such testing, typically different lengths and chemistries of the oligonucleotide will be tested.

In another embodiment, the method is a method of identifying or validating a micro RNA target of a target RNA. Very often, it is hypothesized that a microRNA regulates a given target RNA and in this case, the method of the second aspect is a method of verifying whether the target RNA is indeed regulated by a microRNA. Thus, the method may further comprise identifying the microRNA that regulates the target RNA. This is possible because the target RNA should comprise an anti-seed sequence which is complementary the seed sequence of the microRNA.

### Method of providing an oligonucleotide for blocking the regulatory activity of a microRNA at a target mRNA

A third aspect of the invention is a method of providing an oligonucleotide for blocking the regulatory activity of a microRNA at a target mRNA comprising the steps of:
a. Providing a target sequence of a target mRNA, said target sequence being the sequence of the target mRNA involved in microRNA regulation,
b. Designing an oligonucleotide sequence that comprises a continuous stretch of bases of at least 6 bases that is complementary to the target sequence, and that has a degree of identity to the microRNA of less than 90%, wherein the degree of identity is counted over the length of the shortest molecule of the microRNA and the oligonucleotide, and that is not capable of inducing RNase H cleavage and not capable of recruiting the RNAi machinery, and
c. Synthesizing the oligonucleotide sequence of step b, said oligonucleotide being a candidate regulator of the activity of a target mRNA, wherein the oligonucleotide has reduced off-target effects, as compared to regulating the activity of the target mRNA using an oligonucleotide that can base pair with a microRNA and thereby inhibit the activity of the microRNA and wherein the oligonucleotide comprise nucleotide monomers that increase its affinity for complementary sequences or affinity increasing modifications.
   - wherein the sequence of the microRNA is selected from the group consisting of: SEQ ID NOs: 1-313.

In a preferred embodiment, the method further comprises the steps of Preferably, the method further comprises the steps
a. Providing a reporter system for activity of the target mRNA
b. Determining the activity of the target mRNA in the presence of the candidate regulator
c. Determining the activity of the target RNA in the absence of the candidate regulator
d. Comparing the activity levels in b and c and thereby verifying whether the oligonucleotide is indeed a capable of regulating the activity of the RNA.

Preferably, the method further comprises the steps of:
- providing the sequence of the microRNA regulating the RNA and using the seed sequence of the microRNA to determine an anti-seed sequence of the target sequence and a guide sequence of the oligonucleotide, wherein the guide sequence is a sequence that can base pair to the anti-seed sequence and wherein
- the continuous stretch of bases of the oligonucleotide comprises the guide sequence corresponding to the seed sequence of the microRNA regulating the target RNA, and the target region of the target mRNA comprise the anti-seed sequence complementary to the seed sequence of the microRNA regulating the target RNA.

Preferably, the target sequence of the target mRNA resides within the 3'UTR of the target mRNA.

Also preferably the microRNA and the mRNA is human.

In a preferred embodiment, the length of the continuous stretch of bases complementary to the target sequence is less than 35 bases and more than 7 bases.

Preferably, the oligonucleotide comprises LNA.

It is also preferred that the oligonucleotide does not comprise a stretch of unmodified DNA that exceeds a length of 4 units and does not comprise a stretch of RNA units that exceeds a length of 5.

Moreover, it is preferred that the seed sequence is position 2-7 counted from the 5'end of the microRNA.

Hence, oligonucleotides (candidate regulators) potentially capable of regulating the activity of a target RNA are first identified, where after the activity of these oligonucleotides are tested using a reporter system such as to verify whether the oligonucleotides do indeed have the desired activity, i.e. are capable of regulating the activity of the target RNA.

### Reporter system

The reporter system for expression may be any system that enables a read-out indicative of the activity of the target RNA. It may be e.g. cells harbouring a genetic construct, wherein the target RNA has been fused to another reporter gene.

In a preferred embodiment, the target sequence of the target RNA resides within the 3'-untranslated region of an mRNA. In such cases, the 3'UTR may be fused to a reporter gene without necessarily including the rest of the target mRNA.

The reporter gene may be e.g. the luciferase gene or GFP gene. Such reporter systems are well-known to the skilled man.

The reporter system could also be cells harbouring the endogenous target mRNA. In such an embodiment, the activity (expression) of the target mRNA may be determined by immunoblotting using antibodies targeting the polypeptide or protein encoded by the mRNA. 2D-gel analysis or protein chips may also be used to determine the activity of the target mRNA.

Microarrays, Northern blots and real time PCR (also known as quantitative PCR) may be used to determine any effects on mRNA levels. Also such reporter systems are well known to the skilled man.

In a preferred embodiment, the target region of the target mRNA is comprised within the 3'UTR and comprise a sequence that is complementary to a sequence selected from the group consisting ofposition 2-7of any SEQ ID NOs 1-313.

### References

Ameres SL, M. J. (2007). Molecular basis for target RNA recognition and cleavage by human RISC. Cell, Jul 13;130(1):101-12.
Choi WY, G. A. (2007). Target protectors reveal dampening and balancing of Nodal agonist and antagonist by miR-430. Science, Oct 12;318(5848):271-4. Epub 2007 Aug 30.
Gupta A, G. J. (2006). Anti-apoptotic function of a microRNA encoded by the HSV-1 latency-associated transcript. Nature, Jul 6;442(7098):82-5.
Kawahara Y, Z. B. (2007). Redirection of silencing targets by adenosine-to-inosine editing of miRNAs. Science., Feb 23;315(5815):1137-40.
Kertesz M, I. N. (2007). The role of site accessibility in microRNA target recognition. Nat Genet., Oct;39(10):1278-84. Epub 2007 Sep 23.
Long D, L. R. (2007). Potent effect of target structure on microRNA function. Nat Struct Mol Biol., Apr;14(4):287-94. Epub 2007 Apr 1.
Poy MN, E. L. (2004). A pancreatic islet-specific microRNA regulates insulin secretion. Nature, Nov 11;432(7014), 226-30.
Xiao J, Y. B. (2007). Novel approaches for gene-specific interference via manipulating actions of microRNAs: examination on the pacemaker channel genes HCN2 and HCN4. J Cell Physiol. , Aug;212(2):285-92.

### Exam pies

### Example 1

### A blockmir for treatment of diabetes

It has been demonstrated that mir-375 is a regulator of pancreatic island insulin secretion, and that Myotrophin (*Mtpn*) is a target of mir-375 regulation (Poy MN, 2004). Further, it has been shown that siRNA inhibition of Mtpn mimics the effects of miR-375 on glucose stimulated insulin secretion and exocytosis. Thus, it was concluded that by the authors that miR-375 is a regulator of insulin secretion and may thereby constitute a novel pharmacological target for the treatment of diabetes.

Here we provide blockmirs that can regulate Mtpn expression by inhibiting mir-375 regulation of Mtpn. activity on the 3'UTR of the Mtpn mRNA.

The relevant portion of the target mRNA is:
5'GUGUUUUAAGUUUUGUGUUGCAA**GAACAA**AUGGAAUAAACUUGAAU

The anti-seed sequence is shown in bold. This target region of the target RNA can be identified e.g. by searching the target RNA for anti-seed sequences. Or the target region can be found using suitable databases available on the internet (www.pictar.com, target-scan). Obviously, the information may also be available from experiments or from a scientific publication (as e.g. Poy et al.)

The sequence of mir-375 is:
5'UUUGUUCGUUCGGCUCGCGUGA

Pairing the seed sequence to the anti-seed sequence gives e.g. the following interactions.

5' ...AGUUUGUGUUGC---AAGAACAAAU...
|:|  ||     |||||||
3'AGUGCGC-UCGGCUUGCUUGUUU

It is seen that overall complementarity is scarce.

### The blockmir:

A blockmir capable of regulating Mtpn expression by inhibition of mir-375 regulation will have to be able to sequester the anti-seed sequence of the target region, i.e. hide the anti-seed sequence in base pairing.

Thus, blockmirs (lower strand in 3'-5' direction) of Mtpn are exemplified here, base paired to the target sequence (upper strand in 5'-3' direction):

5′GUGUUUUAAGUUUGUGUUGCAAGAACAAAUGGAAUAAACUUGAAU

| | | | | | | | | | | | | | | | | | | | | | | | | | | | | | | | | |

GUUCAAAACACAACGUUCUUGUUUACCUUAUUUG

5′GUGUUUUAGUUUUGUGUUGCAAGAACAAAUGGAAUAAACUUGAAU
||||||||||||||||||||||||||||
GUUCAAAACACAACGUUCUUGUUUACCUU

5′GUGUUUUAAGUUUUGUGUUGCAAGAACAAAUGGAAUAAACUUGAAU
||||||||||||||||||||||||
GUUCAAAACACAACGUUCUUGUUU

5′ GUGUUUUAGUUUUGUGUUGCAAGAACAAAUGGAAUAAACUUGAAU

| | | | | | | | | | | | | | | | | | | | | | | |

CAAAACACAACGUUCUUGUUUGCC

5′GUGUUUUAAGUUUUGUGUUGCAAGAACAAAUGGAAUAAACUUGAAU

| | | | | | | | | | | | | | | | | | |

CACAACGUUCUUGUUUACC

5′GUGUUUUAGUUUUGUGUUGCAAGAACAAAUGGAAUAAACUUGAAU

|||||||||||||||||

CAACGUUCUUGUUUACC

5′GUGUUUUAAGUUUUGUGUUGCAAGAACAAAUGGAAUAAACUUGAAU
|||||||||||||
CGUUCUUGUUUACC

5′GUGUUUUAGUUUUGUGUUGCAAGAACAAAUGGAAUAAACUUGAAU
|||||||||||||
GUUCUUGUUUACC

5′ GUGUUUUAAGUUUUGUGUUGCAAGAACAAAUGGAAUAAACUUGAAU
| | | | | | | | | | | |
GUUCUUGUUUAC

5′ GUGUUUUAAGUUUUGUGUUGCAAGAACAAAUGGAAUAAACUUGAAU
| | | | | | | | | | |
GUUCUUGUUUA

5′GUGUUUUAAGUUUUGUGUUGCAAGAACAAAUGGAAUAAACUUGAAU
||||||||
CUUGUUUA

The blockmirs designed above may be synthesized using methods known in the art. As described in the specification, they may be synthesised as DNA, RNA, LNA, INA or with mixed monomers.

Obviously, U monomers may be exchanged with T monomers, while still allowing base pairing. Also G-C base pairs may be substituted with G-U wobble base pairs.

Methods for synthesizing various embodiments of the above designed blockmirs targeting the Mtpn mRNA are well known to the skilled man within the field of oligonucleotide synthesis. Particular preferred embodiments are described in the detailed description of the invention.

Conjugation of the blockmirs to e.g. cholesterol is also within the common knowledge of the skilled man.

### Example 2

### A blockmir for treatment of Herpes-simplex virus infection

Recently it was demonstrated that Herpes simplex virus-1 encoded a microRNA that enables the virus to establish a latent infection (Gupta A, 2006). The microRNA that was termed mir-LAT was found to regulate TGF-beta and SMAD3, and thereby affect the ability of the cell to undergo apoptosis, the usual process by which an infected cell self-destructs in order to prevent production of viral progeny. Thus, it is of interest to be able to block the regulatory activity of mir-LAT on the expression of TGF-beta and SMAD3.

The sequence of the target region of the TGF-beta mRNA is:
5'AGGTCCCGCCCCGCCCCGCCCCGCCCCGGCAGGCCCG**GCCCCA**CC

The sequence of mir-LAT is:
5'**UGGCG**GCCCGGCCCGGGGCC

Thus, the following complex may be formed:

5′CGCCCCGGCAGGCCCGGCC-CCA

   |||||||   ||| |||| |||

3'CCGGGGCC---CGGCCCGGCGGU

A series of blockmirs can be designed as was also done in the previous example. The lower strand is the blockmir shown in the 3'-5' direction and the upper strand is the target region of TGF-beta mRNA:

5'AGGUCCCGCCCCGCCCCGCCCCGCCCCGGCAGGCCCGGCCCCACC

|||||||||||||||||||||||||||||||||||||||||||||

UCCAGGGCGGGGCGGGGCGGGGCGGGGCCGUCCGGGCCGGGGUGG

5'AGGUCCCGCCCCGCCCCGCCCCGCCCCGGCAGGCCCGGCCCCACC
||||||||||||||||||||||||||||||||
GGGGCGGGGCGGGGCCGUCCGGGCCGGGGUGG

5'AGGUCCCGCCCCGCCCCGCCCCGCCCCGGCAGGCCCGGCCCCACC
||||||||||||||||||||||
GCGGGGCCGUCCGGGCCGGGGUGG

5'AGGUCCCGCCCCGCCCCGCCCCGCCCCGGCAGGCCCGGCCCCACC

||||||||||||||||||

CCGUCCGGGCCGGGGUGG

5′AGGUCCCGCCCCGCCCCGCCCCGCCCCGGCAGGCCCGGCCCCACC
||||||||||||||
CGGGCCGGGGUGG

5’AGGUCCCGCCCCGCCCCGCCCCGCCCCGGCAGGCCCGGCCCCACC

|||||||||||||

GGCCGGGUGG

5'AGGUCCCGCCCCGCCCCGCCCCGCCCCGGCAGGCCCGGCCCCACC
|||||||||
CCGGGGUGG

5'AGGUCCCGCCCCGCCCCGCCCCGCCCCGGCAGGCCCGGCCCCACC
||||||||
CCGGGGUG

Synthesis of various embodiments of such sequences is well within the ability of the skilled man. Particular preferred embodiments are described in the detailed description of the invention.

### Example 3

Identification of an Oligonucleotide that regulate expression of Mtpn.
The following is a non-limiting example of how the method may be carried out. No wet experiments have actually been carried out.

The following sequence is a portion of the estimated target region of the Mtpn mRNA:

5′UUUGACGCAGUUGGGUUUCUCAUAAGUAUCCUAGUUCAUGUACAUCCGAAUGCUAAAU
AAUACUGUGUUUAAGUUUGUGUUGCAAGAACAAAGGAAUAAACUUGAAUUGUGCUAC

A series of potential blockmirs for this region with a 50% overlap is designed. Potential blockmirs are shown in italic and a reference sequence of the Mtpn mRNA and a complementary strand are shown for comparison:

5′UUUGACGCAGUUGGGUUUCUCAUAAGUAUCCUAGUUCAUGUACAUCCGAAUGCUAAAUAAU-
||||||||||||||||||||||||||||||||||||||||||||||||||||||||||||
3′AAACUGCGUCAACCCAAAGAGUAUUCAUAGGAUCAAGUACAUGUAGGCUUACGAUUUAUUA-

AAACUGCGUCAACCCAAAGA
AACCCAAAGAGUAUUCAUAG
GUAUUCAUAGGAUCAAGUAC
GAUCAAGUACAUGUAGGCUU
AUGUAGGCUUACGAUUUAUUA
ACGAUUUAUUA–

5′-ACUGUGUUUUAAGUUUUGUGUUGCAAGAACAAAUGGAAUAAACUUGAAUUGUGCUAC

| | | | | | | | | | | | | | | | | | | | | | | | | | | | | | | | | | | | | | | | | | | | | | | | | | | | | | | | |

3′-UGACACAAAAUUCAAAACACAACGUUCUUGUUUACCUUAUUUGAACUUAACACGAUG

-UGACACAAAA
UGACACAAAAUUCAAAACAC
UUCAAAACACAACGUUCUUG
AACGUUCUUGUUUACCUUAU
UUUACCUUAUUUGAACUUAA
UUGAACUUAACACGAUG

Thus, 11 blockmirs have been designed. These are then synthesised, e.g. as 2-O-modified oligonucleotides with phosphorothioate linkages. Methods for synthesizing various embodiments of the above designed blockmirs targeting the Mtpn mRNA are well known to the skilled man within the field of oligonucleotide synthesis. Particular preferred embodiments are described in the detailed description of the invention.

Conjugation of the blockmirs to e.g. cholesterol is also within the common knowledge of the skilled man

The target sequence is then fused to a reporter gene, which expression can be detected. In this example, the reporter gene is eGFP. The reporter gene is then transfected to Hela cells, where after expression of eGFP is monitored after transfection of each of the 11 designed blockmirs.

### Result:

Only blockmirs 7-9, counting from blockmirs complementary to the 5'end of the target region, affects the expression of eGFP. Blockmir 7 seven gives a slight increase in eGFP expression, whereas blockmir 8 and 9 has a more dramatic effect.

Thus, oligonucleotides that can affect the expression of the Mtpn mRNA have been identified.

The result indicates that the region covered by blockmirs 7-9 is a target for microRNA regulation. Furthermore, the result indicates that the region covered by blockmirs 8 and 9 is most important for microRNA regulation. The region covered by both oligonucleotide 8 and 9 may comprise the region that interact with the seed sequence of the microRNA or partly comprise the region that interact with the seed sequence of the microRNA.

The region covered by blockmirs 8 and 9 (AAGTTTTGTGTTGCAA**GAACAAA**TGGAATA) is then searched for complementarity to microRNAs.

More specifically, the region is searched for complementarity to seed sequences of microRNA. This search identifies human mir-375.

Whether this microRNA does indeed regulate the activity of the Mtpn mRNA may be verified by inhibiting the activity of mir-375 with an antimir.

The sequence of mir-375 is:
5'UUUGUUCGUUCGGCUCGCGUGA

Thus, an inhibitory antimir with the complementary sequence is synthesized.

Mir-375-antimir:
5'TCACGCGAGCCGAACGAACAAA

Truncated versions of the antimir is also produced:

5′ACGCGAGCCGAACGAACAAA

5′GCGAGCCGAACGAACAAA

5′GAGCCGAACGAACAAA

5′GCCGAACGAACAAA

5′CGAACGAACAAA

The antimirs are synthesised e.g. as 2-modified oligonucleotides with phosphorothioate linkages. Then it is tested whether these antimirs can prevent mir-375 from regulating the Mtpn mRNA using the reporter system.
The result shows that mir-375 does indeed regulate expression of the Mtpn mRNA.

## Claims

1. A method of providing an oligonucleotide for blocking the regulatory activity of a microRNA at a target mRNA comprising the steps of:
a. Providing a target sequence of a target mRNA, said target sequence being the sequence of the target mRNA involved in microRNA regulation,
b. Designing an oligonucleotide sequence that comprises a continuous stretch of bases of at least 6 bases that is complementary to the target sequence, and that has a degree of identity to the microRNA of less than 90%, wherein the degree of identity is counted over the length of the shortest molecule of the microRNA and the oligonucleotide, and that is not capable of inducing RNase H cleavage and not capable of recruiting the RNAi machinery and
c. Synthesizing the oligonucleotide sequence of step b, said oligonucleotide being a candidate regulator of the activity of a target mRNA, wherein the oligonucleotide has reduced off-target effects, as compared to regulating the activity of the target mRNA using an oligonucleotide that can base pair with a microRNA and thereby inhibit the activity of the microRNA and wherein the oligonucleotide comprises nucleotide monomers that increase its affinity for complementary sequences or affinity increasing modifications and
- wherein the sequence of the microRNA is selected from the group consisting of: SEQ ID NOs: 1-313.

2. The method of claim 1 further comprising the steps of:
a. Providing a reporter system for activity of the target mRNA,
b. Determining the activity of the target mRNA in the presence of the oligonucleotide of claim 1 step c,
c. Determining the activity of the target mRNA in the absence of the oligonucleotide of claim 1 step c,
d. Comparing the activity levels in b and c and thereby verifying whether the oligonucleotide is indeed a capable of regulating the activity of the mRNA.

3. The method of any one of the preceding claims further comprising
- providing the sequence of the microRNA regulating the mRNA and using the seed sequence of the microRNA to determine an anti-seed sequence of the target sequence and a guide sequence of the oligonucleotide, wherein the guide sequence is a sequence that can base pair to the anti-seed sequence and wherein
- the continuous stretch of bases of the oligonucleotide comprises the guide sequence corresponding to the seed sequence of the microRNA regulating the target mRNA, and the target sequence of the target mRNA comprises the anti-seed sequence complementary to the seed sequence of the microRNA regulating the target mRNA.

4. The method of any one of the preceding claims, wherein the target sequence of the target mRNA resides within the 3'UTR of the target mRNA.

5. The method of any one of the preceding claims, wherein the microRNA and the mRNA are human.

6. The method of any one of the preceding claims, wherein the length of the continuous stretch of bases complementary to the target sequence is less than 35 bases and more than 7 bases.

7. The method any one of the preceding claims, wherein the oligonucleotide comprises LNA.

8. The method of any one of the preceding claims, wherein the oligonucleotide does not comprise a stretch of unmodified DNA that exceeds a length of 4 units and does not comprise a stretch of RNA units that exceeds a length of 5.

9. The method of any one of the preceding claims, wherein the seed sequence is position 2-7 counted from the 5'end of the microRNA.

10. An oligonucleotide for blocking the regulatory activity of a microRNA at a target mRNA provided by the method of any one of claims 1-9 wherein
- the oligonucleotide comprises a continuous stretch of less than 35 bases and more than 7 bases that is complementary to the target sequence, said target sequence being the sequence of the target mRNA involved in microRNA regulation and
i. wherein said target sequence comprises an anti-seed sequence complementary to the seed sequence of the microRNA regulating the target mRNA,
ii. the seed sequence is position 2-7 counted from the 5'end of the microRNA,
iii. wherein said continuous stretch of bases comprises a guide sequence that can base pair to the anti-seed sequence of the target sequence and,
iv. the target sequence of the target mRNA resides within the 3'UTR of the target mRNA and wherein,
- the oligonucleotide is not capable of inducing RNase H cleavage and not capable of recruiting the RNAi machinery, and
- the oligonucleotide has a degree of identity to the microRNA of less than 90%, wherein the degree of identity is counted over the length of the shortest molecule of the microRNA and the oligonucleotide, and
- the oligonucleotide has reduced off-target effects, as compared to regulating the activity of the target mRNA using an oligonucleotide that can base pair with a microRNA and thereby inhibit the activity of the microRNA and
- the oligonucleotide comprises nucleotide monomers that increase its affinity for complementary sequences or affinity increasing modifications and
- wherein the sequence of the microRNA is selected from the group consisting of: SEQ ID NOs: 1-313.

11. The oligonucleotide of claim 10, wherein one nucleotide monomer that increases affinity for complementary sequences is LNA.

12. An ex vivo or in vitro method of modulating the activity of a target mRNA by preventing the activity of a microRNA at the target mRNA comprising the steps of:
a. Providing a system comprising the target mRNA,
b. Providing an oligonucleotide
c. Introducing the oligonucleotide of step b to the system of step a,
d. Thereby modulating the activity of the target mRNA.
- wherein the oligonucleotide comprises a continuous stretch of less than 35 bases and more than 7 bases that is complementary to the target sequence, said target sequence being the sequence of the target mRNA involved in microRNA regulation and
i. wherein said target sequence comprises an anti-seed sequence complementary to the seed sequence of the microRNA regulating the target RNA,
ii. the seed sequence is position 2-7 counted from the 5'end of the microRNA.
iii. wherein said continuous stretch of bases comprise a guide sequence that can base pair to the anti-seed of the target sequence and,
iv. the target sequence of the target mRNA resides within the 3'UTR of the target mRNA
- the oligonucleotide is not capable of inducing RNase H cleavage and not capable of recruiting the RNAi machinery, and
- the oligonucleotide has a degree of identity to the microRNA of less than 90%, wherein the degree of identity is counted over the length of the shortest molecule of the microRNA and the oligonucleotide, and
- the oligonucleotide has reduced off-target effects, as compared to regulating the activity of the target mRNA using an oligonucleotide that can base pair with a microRNA and thereby inhibit the activity of the microRNA and
- the oligonucleotide comprises nucleotide monomers that increase its affinity for complementary sequences or affinity increasing modifications.
- wherein the sequence of the microRNA is selected from the group consisting of: SEQ ID NOs: 1-313.

13. The method of claim 12, wherein the system is either a cell extract or a cell.

## Patentansprüche

1. Verfahren zur Bereitstellung eines Oligonukleotids zum Blockieren der regulatorischen Aktivität einer mikroRNA an einer Ziel-mRNA, die folgenden Schritte umfassend:
a. Bereitstellen einer Zielsequenz einer Ziel-mRNA, wobei die Zielsequenz die Sequenz der an der mikroRNA-Regulation beteiligten Ziel-mRNA ist,
b. Entwerfen einer Oligonukleotidsequenz, die einen kontinuierlichen Abschnitt von mindestens 6 Basen umfasst, der komplementär zur Zielsequenz ist, und die einen Identitätsgrad zur mikroRNA von weniger als 90% aufweist, wobei der Identitätsgrad über die Länge des kürzesten Moleküls der mikroRNA und des Oligonukleotids gezählt wird, und die nicht in der Lage ist Spaltung durch RNase H zu induzieren und nicht in der Lage ist die RNAi-Maschinerie zu rekrutieren und
c. Synthetisieren der Oligonukleotidsequenz aus Schritt b, wobei das Oligonukleotid ein möglicher Regulator der Aktivität einer Ziel-mRNA ist, wobei das Oligonukleotid reduzierte unspezifische Effekte hat, verglichen mit der Regulierung der Aktivität der Ziel-mRNA unter Verwendung eines Oligonukleotids, das Basenpaarung mit einer mikroRNA eingehen kann und dadurch die Aktivität der mikroRNA hemmt, und wobei das Oligonukleotid Nukleotidmonomere umfasst, die ihre Affinität für komplementäre Sequenzen oder affinitätssteigernde Modifikationen erhöhen und
- wobei die Sequenz der mikroRNA ausgewählt ist aus der Gruppe bestehend aus: SEQ ID Nr: 1-313.

2. Verfahren nach Anspruch 1, ferner die folgenden Schritte umfassend:
a. Bereitstellen eines Reporter-Systems für die Aktivität der Ziel-mRNA,
b. Bestimmen der Aktivität der Ziel-mRNA in Gegenwart des Oligonukleotids nach Anspruch 1 Schritt c,
c. Bestimmen der Aktivität der Ziel-mRNA in Abwesenheit des Oligonukleotids nach Anspruch 1 Schritt c,
d. Vergleichen der Aktivität in b und c und damit Verifizierung, ob das Oligonukleotid tatsächlich in der Lage ist, die Aktivität der mRNA zu regulieren.

3. Verfahren nach einem der vorhergehenden Ansprüche, ferner umfassend
- Bereitstellen der Sequenz der mikroRNA, die die mRNA reguliert und Verwendung der Seed-Sequenz der mikroRNA um eine anti-Seed-Sequenz der Zielsequenz des Oligonukleotids zu bestimmen, wobei die Führungssequenz eine Sequenz ist, die Basenpaarung mit der anti-Seed-Sequenz eingehen kann und wobei
- der kontinuierliche Basen-Abschnitt des Oligonukleotids die Führungssequenz umfasst, die der Seed-Sequenz der mikroRNa entspricht, die die Ziel-mRNA reguliert, und die Zielsequenz der Ziel-mRNA die anti-Seed-Sequenz umfasst, die komplementär zur Seed-Sequenz der mikroRNA ist, die die Ziel-RNA reguliert.

4. Verfahren nach einem der vorhergehenden Ansprüche, wobei die ZielSequenz der Ziel-mRNA innerhalb des 3'-UTR der Ziel-mRNA liegt.

5. Verfahren nach einem der vorhergehenden Ansprüche, wobei die mikroRNA und die mRNA human sind.

6. Verfahren nach einem der vorhergehenden Ansprüche, wobei die Länge des kontinuierlichen Basen-Abschnitts, der komplementär zur Zielsequenz ist, kürzer als 35 Basen und länger als 7 Basen ist.

7. Verfahren nach einem der vorhergehenden Ansprüche, wobei das Oligonukleotid LNA umfasst.

8. Verfahren nach einem der vorhergehenden Ansprüche, wobei das Oligonukleotid keinen Abschnitt unmodifizierter DNA umfasst, der eine Länge von 4 Einheiten überschreitet, und keinen RNA-Abschnitt umfasst, der eine Länge von 5 überschreitet.

9. Verfahren nach einem der vorhergehenden Ansprüche, wobei die Seed-sequenz vom 5'-Ende der mikroRNA gezählt Position 2-7 hat.

10. Oligonukleotid zum Blockieren der regulatorischen Aktivität einer mikroRNA an einer Ziel-mRNA, die durch das Verfahren nach einem der Ansprüche 1-9 bereitgestellt wird, wobei
- das Oligonukleotid einen kontinuierlichen Abschnitt von weniger als 35 Basen und mehr als 7 Basen umfasst, der zur Zielsequenz komplementär ist, wobei die Zielsequenz die Sequenz der Ziel-mRNA ist, die an der mikroRNA-Regulation beteiligt ist, und
i. wobei die Zielsequenz eine anti-Seed-Sequenz umfasst, die komplementär zur Seed-Sequenz der mikroRNA ist, die die Ziel-mRNA reguliert,
ii. die Seed-Sequenz vom 5'-Ende der mikroRNA gezählt Position 2-7 hat,
iii. wobei der kontinuierliche Basen-Abschnitt eine Führungssequenz umfasst, die Basenpaarung mit der anti-Seed-Sequenz der Zielsequenz eingehen kann und,
iv. die Zielsequenz der Ziel-mRNA innerhalb des 3'-UTR der Ziel-mRNA liegt und worin,
- das Oligonukleotid nicht in der Lage ist Spaltung durch RNase H zu induzieren und nicht in der Lage ist die RNAi-Maschinerie zu rekrutieren, und
- das Oligonukleotid einen Identitätsgrad zur mikroRNA von weniger als 90% aufweist, wobei der Identitätsgrad über die Länge des kürzesten Moleküls der mikroRNA und des Oligonukleotids gezählt wird, und
- das Oligonukleotid reduzierte unspezifische Effekte hat, verglichen mit der Regulierung der Aktivität der Ziel-mRNA unter Verwendung eines Oligonukleotids, das Basenpaarung mit einer mikroRNA eingehen kann und dadurch die Aktivität der mikroRNA hemmt und
- das Oligonukleotid Nukleotidmonomere umfasst, die seine Affinität für komplementäre Sequenzen oder affinitätssteigernde Modifikationen erhöhen und
- wobei die Sequenz der mikroRNA ausgewählt ist aus der Gruppe bestehend aus: SEQ ID Nr: 1-313.

11. Oligonukleotid nach Anspruch 10, wobei ein Nukleotidmonomer, das die Affinität für komplementäre Sequenzen erhöht, LNA ist.

12. Ex vivo oder in vitro Verfahren zur Modulation der Aktivität einer Ziel-mRNA durch Verhindern der Aktivität einer mikroRNA an der Ziel-RNA, umfassend die folgenden Schritte:
a. Bereitstellen eines Systems, das die Ziel-mRNA umfasst,
b. Bereitstellen eines Oligonukleotids
c. Einführen des Oligonukleotids aus Schritt b in das System aus Schritt a,
d. wodurch die Aktivität der Ziel-mRNA moduliert wird
- wobei das Oligonukleotid einen kontinuierlichen Abschnitt von weniger als 35 Basen und mehr als 7 Basen umfasst, der komplementär zur Zielsequenz ist, wobei die Ziel-Sequenz die Sequenz der an der mikroRNA-Regulation beteiligten Ziel-mRNA ist und
i. wobei die Ziel-Sequenz eine anti-Seed-Sequenz umfasst, die komplementär ist zur Seed-Sequenz der mikroRNA, die die Ziel-RNA reguliert,
ii. die Seed-Sequenz vom 5'-Ende der mikroRNA gezählt Position 2-7 hat,
iii. wobei der kontinuierliche Basen-Abschnitt eine Führungssequenz umfasst, die Basenpaarung mit der anti-Seed-Sequenz der Zielsequenz eingehen kann und,
iv. die Zielsequenz der Ziel-mRNA innerhalb des 3'-UTR der Ziel-mRNA liegt
- das Oligonukleotid nicht in der Lage ist Spaltung durch RNase H zu induzieren und nicht in der Lage ist die RNAi-Maschinerie zu rekrutieren, und
- das Oligonukleotid einen Identitätsgrad zur mikroRNA von weniger als 90% aufweist, wobei der Identitätsgrad über die Länge des kürzesten Moleküls der mikroRNA und des Oligonukleotids gezählt wird, und
- das Oligonukleotid reduzierte unspezifische Effekte hat, verglichen mit der Regulierung der Aktivität der Ziel-mRNA unter Verwendung eines Oligonukleotids, das Basenpaarung mit einer mikroRNA eingehen kann und dadurch die Aktivität der mikroRNA hemmt und
- das Oligonukleotid Nukleotidmonomere umfasst, die seine Affinität für komplementäre Sequenzen oder affinitätssteigernde Modifikationen erhöhen und
- wobei die Sequenz der mikroRNA ausgewählt ist aus der Gruppe bestehend aus: SEQ ID Nr: 1-313.

13. Verfahren nach Anspruch 12, wobei das System entweder ein Zellextrakt oder eine Zelle ist.

## Revendications

1. Procédé de fourniture d'un oligonucléotide pour bloquer l'activité de régulation d'un microARN à un ARNm cible comprenant les étapes de:
a. fournir une séquence cible d'un ARNm cible, ladite séquence cible étant la séquence de l'ARNm cible impliqué dans la régulation microARN,
b. concevoir une séquence oligonucléotidique qui comprend une séquence continued'au moins 6 bases qui est complémentaire de la séquence cible, et qui a un degré d'identité au microARN inférieur à 90%, dans lequel le degré d'identité est compté sur la longueur de la molécule la plus courte du microARN et de l'oligonucléotide, et qui n'est pas capable d'induire la clivage par RNase H et pas capable de recruter les machines ARNi et
c. synthétiser la séquence oligonucléotidique de l'étape b, leditoligonucléotide étant un régulateur candidat de l'activité d'un ARNm cible, l'oligonucléotide ayant des effets hors cible réduits, comparé à la régulation de l'activité de l'ARNm cible en utilisant un oligonucléotide qui peut être apparier de base à un microARN et ainsi inhiber l'activité du microARN et dans lequel l'oligonucléotide comprend des monomères nucléotidiques qui augmentent son affinité pour des séquences complémentaires ou des modifications augmentant l'affinité et
- dans lequel la séquence du microARN est choisie dans le groupe consistant de: SEQ ID NOs: 1-313.

2. Procédé selon la revendication 1 comprenant en outre les étapes de:
a. fournir un système rapporteur pour l'activité de l'ARNm cible,
b. déterminer l'activité de l'ARNm cible en présence de l'oligonucléotide selon la revendication 1, étape c,
c. déterminer l'activité de l'ARNm cible en l'absence de l'oligonucléotide selon la revendication 1, étape c,
d. comparer les niveaux d'activité en b et c et vérifier ainsi si l'oligonucléotide est en effet capable de réguler la activité de l'ARNm.

3. Procédé selon l'une quelconque des revendications précédentes, comprenant en outre
- fournissant la séquence du microARN régulant l'ARNm et utilisant la séquence de graine du microARN pour déterminer un séquence anti-graine de la séquence cible et une séquence de guidage de l'oligonucléotide, dans lequel la séquence de guidage est une séquence qui peut apparier de base à la séquence anti-graine et dans laquelle
- la séquence continu des bases de l'oligonucléotide comprend la séquence de guidage correspondant à la séquence de graine de la microARN régulant l'ARNm cible, et la séquence cible de l'ARNm cible comprend la séquence anti-graine complémentaire à la séquence de graine du microARN régulant l'ARNm cible.

4. Procédé selon l'une quelconque des revendications précédentes, dans lequel la séquence cible de l'ARNm cible réside dans le 3'UTR de l'ARNm cible.

5. Procédé selon l'une quelconque des revendications précédentes, dans lequel le microARN et l'ARNm sont humains.

6. Procédé selon l'une quelconque des revendications précédentes, dans lequel la longueur de la séquence continu de bases complémentaires de la séquence cible est moins que 35 bases et plus de 7 bases.

7. Procédé selon l'une quelconque des revendications précédentes, dans lequel l'oligonucléotide comprend du LNA.

8. Procédé selon l'une quelconque des revendications précédentes, dans lequel l'oligonucléotide ne comprend pas une séquence d'ADN non modifié qui dépasse une longueur de 4 unités et ne comprend pas de séquence d'unités d'ARN qui dépasse une longueur de 5.

9. Procédé selon l'une quelconque des revendications précédentes, dans lequel la séquence de graine est la position 2-7 comptée à partir de l'extrémité 5' du microARN.

10. Oligonucléotide pour bloquer l'activité de régulation d'un microARN à un ARNm cible fourni par le procédé de l'une quelconque des revendications 1 à 9, dans lequel
- l'oligonucléotide comprend une séquence continu de moins de 35bases et plus de 7 bases qui est complémentaire de la séquence cible, ladite séquence cible étant la séquence cible de l'ARNm impliqué dans la régulation de microARN et
i. dans lequel ladite séquence cible comprend un séquence anti-graine complémentaire de la séquence de graine de la microARN régulant l'ARNm cible,
ii. la séquence de graine est la position 2-7 comptée à partir de l'extrémité 5' de le microARN,
iii. dans lequel ledit séquence continu de bases comprend une séquence de guidage qui peut apparier de base à la séquence anti-graine de la séquence cible et,
iv. la séquence cible de l'ARNm cible réside dans le 3'UTR de l'ARNm cible et dans lequel,
- l'oligonucléotide n'est pas capable d'induire le clivage de la RNase H et incapable de recruter le mécanisme de l'ARNi, et
- l'oligonucléotide a un degré d'identité avec le microARN inférieur à 90%, le degré d'identité étant compté sur la longueur de la molécule la plus courte du microARN et de l'oligonucléotide, et
- l'oligonucléotide a les effets hors cible réduits, comparé à la régulation de l'activité de l'ARNm cible en utilisant un oligonucléotide qui peut apparier de base à un microARN et ainsi inhiber l'activité du microARN et
- l'oligonucléotide comprend des monomères nucléotidiques qui augmentent l'affinité pour des séquences complémentaires ou des modifications augmentant l'affinité et
- dans lequel la séquence du microARN est sélectionnée dans le groupe consistant de: SEQ ID NOs: 1-313.

11. Oligonucléotide de la revendication 10, dans lequel un monomère nucléotidique qui augmente l'affinité pour les séquences complémentaires est LNA.

12. Procédé ex vivo ou in vitro de modulation de l'activité d'un ARNm cible en inhibant l'activité d'un microARN à l'ARNm cible comprenant les étapes de:
a. fournir un système comprenant l'ARNm cible,
b. fournir un oligonucléotide
c. introduire l'oligonucléotide de l'étape b dans le système de l'étape a,
d. modulant ainsi l'activité de l'ARNm cible.
- dans lequel l'oligonucléotide comprend une séquence continu de moins que 35 bases et plus de 7 bases qui est complémentaire à la séquence cible, ladite séquence cible étant la séquence de ARNm cible impliqué dans la régulation de microARN et
i. dans lequel ladite séquence cible comprend une séquence anti-graine complémentaire de la séquence de graine de la microARN régulant l'ARN cible,
ii. la séquence de graine est la position 2-7 comptée à partir de l'extrémité 5' du microARN.
iii. dans lequel ladite séquence continu de bases comprend une séquence de guidage qui peut apparier de base à l'anti-graine de la séquence cible et,
iv. la séquence cible de l'ARNm cible réside dans le 3'UTR de l'ARNm cible
- l'oligonucléotide n'est pas capable d'induire le clivage de la RNase H et n'est pas capable de recruter la machinerie de l'ARNi, et
- l'oligonucléotide a un degré d'identité avec le microARN inférieur à 90%, dans lequel le degré d'identité est compté sur la longueur de la molécule la plus courte du microARN et de l'oligonucléotide, et
- l'oligonucléotide a des effets hors cible réduits, comparé à la régulation de l'activité de l'ARNm cible en utilisant un oligonucléotide qui peut s'apparier de bases à un microARN et ainsi inhiber l'activité du microARN et
- l'oligonucléotide comprend des monomères nucléotidiques qui augmentent son affinité pour des séquences complémentaires ou des modifications augmentant l'affinité.
- dans lequel la séquence du microARN est choisie dans le groupe consistant de: SEQ ID NOs: 1-313.

13. Procédé selon la revendication 12, dans lequel le système est soit un extrait cellulaire, soit une cellule.
